(19) Europäisches Patentamt
European Patent Office
Office européen des brevets

(11) **EP 3 888 748 A1**

(12) **DEMANDE DE BREVET EUROPEEN**

(43) Date de publication:
**06.10.2021 Bulletin 2021/40**

(21) Numéro de dépôt: **21165904.0**

(22) Date de dépôt: **30.03.2021**

(51) Int Cl.:
*A61N 7/00* (2006.01)    *A61N 7/02* (2006.01)
*B06B 1/00* (2006.01)    *G10K 11/34* (2006.01)
*B06B 1/02* (2006.01)    *B06B 1/06* (2006.01)
*G10K 11/32* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **02.04.2020 FR 2003282**

(71) Demandeurs:
• **EDAP TMS France**
  **69120 Vaulx-en-Velin (FR)**
• **INSTITUT NATIONAL DE LA SANTE ET DE LA RECHERCHE MEDICALE - INSERM**
  **75013 Paris (FR)**

• **Université Claude Bernard Lyon I**
  **69100 Villeurbanne (FR)**
• **Centre Léon Bérard**
  **69008 Lyon (FR)**

(72) Inventeurs:
• **SANCHEZ, Marine**
  **69003 Lyon (FR)**
• **MELO DE LIMA, David**
  **01600 Saint-Bernard (FR)**
• **VINCENOT, Jérémy**
  **69100 Villeurbanne (FR)**

(74) Mandataire: **Cabinet Beau de Loménie**
  **51 avenue Jean Jaurès**
  **BP 7073**
  **69301 Lyon Cedex 07 (FR)**

(54) **APPAREIL DE THERAPIE POUR LE TRAITEMENT DE TISSUS PAR L'EMISSION D'ONDES ULTRASONORES FOCALISEES CROISEES DEPORTEES**

(57) L'invention concerne un appareil de thérapie pour le traitement de tissus par l'émission d'ondes ultrasonores focalisées, comportant :
- une surface de création d'un champ de pression d'ondes ultrasonores focalisées divisée en au moins N secteurs présentant des segments de courbe concave (S1, S2, ...) asymétriques avec des centres de courbure ;
- des centres de courbure ($c_1$, $c_2$, ...) asymétriques dans la mesure où les centres de courbure sont situés à des distances différentes du plan de symétrie (A1) ou de l'axe de symétrie (S) et/ou à des profondeurs différentes prises selon l'axe de symétrie ;
- les axes propres ($a_1$, $a_2$, ...) se coupant entre les zones focales ($Zc_1$, $Zc_2$, ...) et la surface de création (8) ou au-delà des zones focales de manière que les faisceaux issus des secteurs se croisent pour créer une zone focale de recouvrement (Zr) qui est désaxée par rapport au plan de symétrie (A1) ou à l'axe de symétrie (S) ;
- les secteurs de cette surface de création (8) créant des zones de dépôt d'énergie avec des profils correspondant aux zones focales de recouvrement (Zr).

[Fig. 4]

## Description

Domaine Technique

La présente invention concerne le domaine technique des appareils ou des dispositifs comportant une sonde ultrasonore formée par une pluralité d'éléments transducteurs ultrasonores, adaptés pour émettre des ultrasons focalisés de haute intensité (HIFU).

L'objet de la présente invention trouve des applications particulièrement avantageuses dans le domaine des traitements thérapeutiques par des ondes ultrasonores focalisées.

Technique antérieure

Il est connu notamment par la publication, « Kennedy, J.E., High-intensity focused ultrasound in the treatment of solid tumours. Nat Rev Cancer, 2005. 5(4): p. 321-7 » que le traitement par des ondes ultrasonores focalisées permet de créer des lésions biologiques dans les tissus résultant d'une combinaison des effets thermiques et de l'activité de cavitation acoustique. La mise en œuvre d'un tel traitement nécessite la maîtrise de l'énergie déposée et la réalisation en un minimum de temps, d'une lésion de taille appropriée aux tissus à traiter. Par ailleurs, ce traitement doit être envisagé avec le moins de déplacements possibles de la sonde car les déplacements introduisent une sensibilité aux mouvements, impliquent une augmentation des temps de traitement mais aussi une imprécision due à la manipulation manuelle ou alors imposent l'introduction d'un système robotisé pour permettre des déplacements millimétriques. De plus, selon les tissus à traiter, les déplacements de la sonde ne sont pas toujours envisageables car les fenêtres acoustiques disponibles pour traiter les tissus sont parfois très restreintes (Cf. « Aubry, J.F., et al., The road to clinical use of high-intensity focused ultrasound for liver cancer : technical and clinical consensus. J Ther Ultrasound, 2013. 1: p. 13 »).

La forme de ces lésions tissulaires est issue directement de la forme de la surface d'émission de la sonde ultrasonore utilisée. Par exemple, la focalisation géométrique naturelle d'un transducteur ultrasonore HIFU sphérique classique est ellipsoïdale du fait de la diffraction. Aussi, l'état de la technique a proposé diverses solutions pour augmenter la taille de la zone traitée, sans avoir à déplacer la sonde ultrasonore.

Le brevet EP 0 214 782 décrit une solution relative à un transducteur ultrasonore équipé d'une lentille permettant d'obtenir une focalisation annulaire à partir d'un transducteur de géométrie sphérique. Cette construction particulière permet d'élargir la zone focale à la taille de l'anneau mais fait également apparaître une zone focale de recouvrement située sur l'axe central du transducteur issu du croisement des faisceaux ultrasonores au-delà du plan de focalisation du transducteur. Ce document prévoit un système pour réduire le champ de pression dans cette zone focale de recouvrement afin de s'affranchir du risque de lésions secondaires.

Le document WO 2011/092683 décrit une sonde à ultrasons focalisés à haute intensité comprenant un premier réseau de transducteurs ultrasonores pour focaliser un premier faisceau sur un volume cible situé dans une lésion et un deuxième réseau de transducteurs ultrasonores pour produire un deuxième faisceau ultrasonore focalisé sur le même volume cible de telle sorte que l'interférence entre lesdits premier et second faisceaux crée dans ledit volume cible un champ ultrasonore asymétrique. Ce document décrit diverses formes de réalisation de la sonde. Le principal inconvénient est que les deux faisceaux ultrasonores sont générés avec des fréquences différentes, ceci requiert donc une électronique plus couteuse, des calculs de déphasages plus complexes et entraine l'apparition de lobes secondaires de pression en dehors du volume cible.

Il est à noter qu'une sonde de traitement par ultrasons peut être utilisée en association avec une sonde d'imagerie comme proposé par le brevet EP 0 661 029. Dans le même sens, le brevet US 5 522 869 décrit un appareil de traitement par énergie ultrasonore comportant une sonde cylindrique avec des transducteurs sous forme d'anneaux.

Le document XP 55009820 décrit une sonde de thérapie comportant une face d'émission de révolution engendrée par la rotation autour d'un axe de symétrie, d'un segment de courbe concave avec le centre de courbure qui se trouve à distance de l'axe de symétrie. Cette face d'émission présente dans un plan de profil, deux segments de courbe concave symétriques, par rapport à l'axe de symétrie, avec chaque segment de courbe concave possédant un axe acoustique passant par le centre de courbure et le milieu du segment de courbe concave. Un tel transducteur permet d'obtenir d'une part, un anneau de focalisation des ondes ultrasonores délimitées par le plan focal et d'autre part, une zone de croisement des faisceaux ultrasonores. Comme il ressort clairement de ce document, la zone de croisement des faisceaux ultrasonores qui correspond au pic secondaire de pression est située derrière le plan focal.

Le brevet EP 2 035 091 décrit un transducteur HIFU de forme torique conduisant à l'obtention d'une zone de focalisation en forme d'un anneau au centre duquel est situé le point central de focalisation.

Le brevet EP 2 691 154 décrit une sonde de thérapie comportant une face d'émission dont les émetteurs ultrasonores sont adaptés pour créer une première zone focale s'établissant dans un plan focal et une deuxième zone focale qui est localisée et située entre la première zone focale et la face d'émission, cette deuxième zone focale correspondant à une zone de recouvrement des faisceaux ultrasonores.

Le document « Vincenot, J., et al., Electronic beam steering used with a toroidal HIFU transducer substantially increases the coagulated volume. Ultrasound Med Biol, 2013. 39(7) : p. 1241-54 » décrit la technique de focalisation électronique permettant d'introduire un léger champ de liberté selon l'axe perpendiculaire à l'axe acoustique, conduisant ainsi à déplacer la lésion à quelques millimètres selon cet axe. Cette solution permet de traiter des zones de grands volumes (50cm3) en un minimum de temps (2 minutes).

Le document WO 2011/024074 propose une solution pour remédier au nombre important d'éléments émetteurs et de générateurs de courant mis en œuvre dans la technique de focalisation électronique qui classiquement est coûteuse et complexe. Cette solution vise à regrouper les éléments émetteurs par paquets afin qu'un générateur de courant puisse en alimenter plusieurs d'entre eux en parallèle, permettant ainsi de réduire le nombre de générateurs de courant. De tels regroupements sont modifiables électroniquement de manière à permettre un déplacement du point focal adapté à la forme des zones à traiter. Si cette solution permet de réduire le coût de l'électronique de commande, elle ne permet pas de réduire le coût du transducteur qui comporte plusieurs centaines d'éléments émetteurs. La fabrication d'un tel transducteur demande l'utilisation d'outils numériques de micro usinage et nécessite une découpe multiéléments difficilement réalisable sur un transducteur en céramique classique notamment à cause des problèmes de couplage électro-acoustique entre les éléments.

Il est également connu par la demande de brevet WO 2016/144931, un système et un procédé d'émission d'ondes ultrasonores pour la régénération des tissus mettant en œuvre un cycle de balayage déterminé.

L'analyse des différentes solutions de l'art antérieur conduit à considérer que le traitement d'une zone de tissus présentant des configurations complexes s'écartant de l'axe acoustique présente des difficultés. En effet, le traitement par des ondes ultrasonores focalisées au sens large du terme, d'une configuration complexe demande un grand nombre d'éléments (et donc une électronique associée) et un temps de traitement long. Dans le cas du traitement par des ondes ultrasonores focalisées par un transducteur torique, la pression reste maximale le long de l'axe acoustique, ce qui implique une difficulté à élargir la zone traitement par rapport à l'axe acoustique et à traiter des configurations complexes qui ne présentent pas une symétrie de révolution.

Tel est le cas notamment, des tissus à traiter dans le cadre d'un cancer du sein. La forme conique de cet organe présentant l'air aréolaire à son sommet associé à des tailles disparates contraint le plus souvent à une unique position possible pour la sonde afin de traiter la tumeur. C'est à partir de cette unique position que doit être traitée la totalité de la zone cible comprenant la tumeur mais aussi une certaine quantité de marges saines, ce qui implique la nécessité de venir déposer l'énergie en profondeur mais aussi de déposer cette énergie de telle façon que la lésion finale soit autant étendue selon l'axe perpendiculaire à l'axe acoustique que dans cet axe lui-même.

Tel est le cas aussi pour une zone cible présentant un volume non symétrique le long de l'axe acoustique et avec une distance selon l'axe perpendiculaire à l'axe acoustique qui évolue le long de l'axe acoustique. Cette zone cible peut par exemple s'adapter à une tumeur de forme complexe ou un organe de forme complexe (prostate, rein, thyroïde...). Une autre configuration de zones cibles concerne un volume creux s'étendant autour d'un segment non rectiligne permettant par exemple le traitement de tumeurs le long des artères ou veines, le long du tube digestif, le long des os, le long du canal du Wirsung dans le pancréas, l'urètre ou le canal biliaire.

La présente invention vise à remédier aux inconvénients des diverses solutions techniques antérieures en proposant un nouvel appareil de thérapie de faible coût, adapté pour permettre d'obtenir un volume de lésions biologiques adapté à des configurations complexes de tissus à traiter alors que la fenêtre acoustique de traitement est particulièrement restreinte.

Pour atteindre un tel objectif, l'appareil de thérapie pour le traitement de tissus par l'émission d'ondes ultrasonores focalisées comporte une sonde de thérapie présentant un transducteur comportant une pluralité d'émetteurs ultrasonores activés par des signaux délivrés par un générateur de signal faisant partie d'un circuit de commande, pour définir une surface de création d'un champ de pression d'ondes ultrasonores focalisées.

Selon l'invention :

- la surface de création est divisée en au moins N secteurs (avec N compris entre 2 et 32) pour focaliser les ondes ultrasonores sur des zones focales s'établissant dans des plans focaux, les secteurs de cette surface de création présentant dans un plan de profil, des segments de courbe concave de longueur finie, asymétriques par rapport à un plan de symétrie ou un axe de symétrie ;
- les centres de courbure sont asymétriques par rapport au plan ou à l'axe de symétrie dans la mesure où les centres de courbure sont situés à des distances différentes du plan de symétrie ou de l'axe de symétrie et/ou à des profondeurs différentes prises selon l'axe de symétrie ;
- chaque segment de courbe concave possède un axe propre passant par un centre de courbure dudit segment de courbe concave et le milieu dudit segment de courbe concave ;
- les axes propres se coupent entre les zones focales et la surface de création ou au-delà des zones focales de manière que les faisceaux issus des secteurs se croisent pour créer une zone focale de recouvrement qui est désaxée par rapport au plan de symétrie ou à l'axe de symétrie et située à distance des plans focaux, entre les zones focales et la surface de création ou au-delà des zones focales ;

- les secteurs de cette surface de création sont engendrés soit par la rotation de 2n/N des segments de courbe concave autour de l'axe de symétrie soit par la translation des segments de courbe selon une direction perpendiculaire au plan de profil contenant lesdits segments de courbe de manière que les secteurs puissent créer des zones de dépôt d'énergie avec des profils correspondant aux zones focales de recouvrement ;
- les segments de courbe de la surface de création s'étendent dans le plan de profil de part et d'autre de l'axe de symétrie ou du plan de symétrie, en étant disjoints pour permettre le positionnement de la zone focale de recouvrement à distance de la surface de création.

Selon une caractéristique préférée de réalisation, la surface de création est divisée en deux secteurs.

Avantageusement, les bords internes délimitent dans la surface de création, un logement pour le montage d'une sonde d'imagerie ultrasonore.

Selon une première variante de réalisation, la surface de création est issue d'une face définie par les éléments transducteurs, de géométrie torique engendrée par la rotation des segments de courbe concave autour de l'axe de symétrie de sorte que les segments de courbe concave suivent des arcs de cercles non coïncidents qui se coupent de sorte que les zones focales possèdent une forme en portions de cercle.

Selon une caractéristique de réalisation, la face est tronquée de manière symétrique par rapport à l'axe de symétrie.

Selon une deuxième variante de réalisation, la surface de création est issue d'une face définie par les éléments transducteurs, de géométrie cylindrique engendrée par la translation selon une longueur limitée, des deux segments de courbe selon une direction perpendiculaire au plan de profil contenant lesdits segments de courbe de sorte que les zones focales possèdent une forme linéaire.

Selon un mode de réalisation, les émetteurs ultrasonores du transducteur définissent une face d'émission correspondant à la surface de création d'un champ de pression d'ondes ultrasonores focalisées.

Selon un autre mode de réalisation, le générateur de signal faisant partie du circuit de commande est piloté pour délivrer des signaux pour activer les émetteurs ultrasonores répartis en segments, avec une loi de retards ou de phases afin de réaliser la surface de création d'un champ de pression d'ondes ultrasonores focalisées.

Selon une variante avantageuse de réalisation, dans une phase d'exposition, les émetteurs ultrasonores faisant partie d'un secteur et du secteur symétriquement opposé par rapport à l'axe de symétrie, sont activés par des signaux délivrés par le générateur de signal faisant partie du circuit de commande pour créer la zone de dépôt d'énergie correspondante.

Selon une caractéristique de l'invention, pour dans une phase d'exposition, les émetteurs ultrasonores faisant partie d'un secteur et du secteur symétriquement opposé par rapport au plan de symétrie sont activés par des signaux délivrés par le générateur de signal faisant partie du circuit de commande pour créer la zone de dépôt d'énergie correspondante d'un côté du plan de symétrie

Selon une autre caractéristique de l'invention, dans une phase d'exposition subséquente, les émetteurs ultrasonores faisant partie d'un secteur et du secteur symétriquement opposé par rapport au plan de symétrie sont activés par des signaux délivrés par le générateur de signal faisant partie du circuit de commande pour créer la zone de dépôt d'énergie correspondante du côté opposé au côté dans lequel est créé la zone de dépôt d'énergie de la phase d'exposition précédente.

Avantageusement, les émetteurs ultrasonores sont répartis selon plusieurs secteurs perpendiculaires au plan de symétrie et les émetteurs ultrasonores des secteurs dans des phases d'exposition successives sont activés par des signaux délivrés par le générateur de signal faisant partie du circuit de commande, pour créer des zones de dépôt d'énergie de part et d'autre du plan de symétrie.

Dans le cadre de l'invention, les émetteurs ultrasonores dans des phases d'exposition successives pour chacune desquelles les centres de courbure sont situés à des distances différentes du plan de symétrie ou de l'axe de symétrie et/ou à des profondeurs différentes selon l'axe vertical sont activés par des signaux délivrés par le générateur de signal faisant partie du circuit de commande de manière à obtenir des zones de dépôt d'énergie désaxées.

Selon un mode de mise en œuvre, les émetteurs ultrasonores dans des phases d'exposition successives pour lesquelles les centres de courbure sont situés à des distances différentes du plan de symétrie ou de l'axe de symétrie et/ou à des profondeurs différentes selon l'axe vertical sont activés par des signaux délivrés par le générateur de signal faisant partie du circuit de commande de manière à obtenir pour ces phases d'exposition successives, des zones de dépôt d'énergie désaxées de positions différentes avec des tailles identiques ou différentes.

Selon un autre mode de mise en œuvre, pour, dans des phases d'exposition successives, les émetteurs ultrasonores sont activés par des signaux délivrés par le générateur de signal faisant partie du circuit de commande de manière que les distances et/ou les profondeurs des centres de courbure sont modifiées d'une phase d'exposition à l'autre afin que les zones de dépôt d'énergie soient concentriques et/ou symétriques et/ou asymétriques et/ou superposées selon l'axe vertical.

Selon un mode complémentaire de mise en œuvre, dans au moins une phase d'exposition complémentaire, les émetteurs ultrasonores sont activés par des signaux délivrés par le générateur de signal faisant partie du circuit de commande de manière à assurer la focalisation des ondes ultrasonores dans des zones focales et à obtenir une zone focale de recouvrement centrée par rapport au plan de symétrie ou à l'axe de symétrie et située à distance des plans focaux entre les zones focales et la face d'émission ou au-delà des zones focales.

L'invention sera mieux comprise à partir de la description détaillée qui va suivre, par référence aux Figures annexées.

Brève description des dessins

[Fig. 1]La Figure 1 est un schéma de principe d'un appareil de thérapie comportant une sonde de thérapie illustrée selon une première variante de réalisation, avec une forme de révolution autour d'un axe de symétrie.

[Fig. 2]La Figure 2 est une vue schématique montrant un exemple d'une surface de création d'un champ de pression d'ondes ultrasonores focalisées réalisée selon la première variante de réalisation et, considérée comme une surface de création virtuelle.

[Fig. 3]La Figure 3 est une vue explicitant le principe pour réaliser la surface de création d'un champ de pression d'ondes ultrasonores focalisées, illustrée à la Fig. 2.

[Fig. 4]La Figure 4 est une coupe élévation dans le plan de profil, d'un premier exemple de la surface de création d'un champ de pression d'ondes ultrasonores focalisées, pouvant être mis en oeuvre selon la première variante de réalisation de la sonde et selon une deuxième variante de réalisation, avec une forme pseudo-cylindrique.

[Fig. 5]La Figure 5 est une coupe élévation dans le plan de profil, d'un deuxième exemple de la surface de création d'un champ de pression d'ondes ultrasonores focalisées, pouvant être mis en œuvre selon la première variante de réalisation et selon la deuxième variante de réalisation de la sonde.

[Fig. 6]La Figure 6 est une coupe élévation dans le plan de profil, d'un troisième exemple de la surface de création d'un champ de pression d'ondes ultrasonores focalisées, pouvant être mis en œuvre selon la première variante de réalisation et selon la deuxième variante de réalisation de la sonde.

[Fig. 6A]La Figure 6A est une coupe élévation dans le plan de profil, d'un quatrième exemple de la surface de création d'un champ de pression d'ondes ultrasonores focalisées, pouvant être mis en œuvre selon la première variante de réalisation et selon la deuxième variante de réalisation de la sonde.

[Fig. 7A-7C]La Figure 7A est une vue face arrière d'une sonde réalisée selon la première variante de réalisation et illustrant le découpage en quatre secteurs de la face de la sonde. La Figure 7B est une vue illustrant une surface de création d'un champ de pression d'ondes ultrasonores focalisées, crée par la sonde illustrée à la Fig. 7A. La Figure 7C est une vue illustrant une surface de création d'un champ de pression d'ondes ultrasonores focalisées, crée par la sonde illustrée à la Fig. 7A.

[Fig. 8A]La Figure 8A est une vue de profil d'une sonde réalisée selon la première variante de réalisation se présentant sous la forme d'un transducteur torique avec une ouverture centrale.

[Fig. 8B]La Figure 8B est une vue en coupe élévation illustrant le principe de focalisation naturelle centrée mis en œuvre par une sonde réalisé selon la première variante de réalisation se présentant sous la forme d'un transducteur torique illustré à la Fig. 8A.

[Fig. 8C]La Figure 8C est une vue en coupe prise perpendiculairement à l'axe de symétrie selon un plan Pr et illustrant la forme de la zone focale de recouvrement des faisceaux ultrasonores en son centre, obtenue selon le principe de focalisation naturelle centrée illustré à la Fig. 8B.

[Fig. 9A]La Figure 9A est une vue schématique montrant un exemple de réalisation d'une surface virtuelle de création d'un champ de pression d'ondes ultrasonores focalisées, obtenue par un transducteur réalisé selon la première variante de réalisation et tel qu'illustré à la Fig. 8A.

[Fig. 9B]La Figure 9B est une vue en coupe élévation dans le plan de profil, illustrant le principe de focalisation par le transducteur illustré à la Fig. 8A pour réaliser une surface virtuelle de création d'un champ de pression d'ondes ultrasonores focalisées, montrée à la Fig. 9A de manière à obtenir une zone focale de recouvrement désaxée des faisceaux ultrasonores illustrée par rapport à la zone focale de recouvrement des faisceaux ultrasonores, obtenue selon le principe de focalisation naturelle.

[Fig. 9C]La Figure 9C est une vue en coupe prise perpendiculairement à l'axe de symétrie selon un plan Pr et illustrant la forme de la zone focale de recouvrement désaxée des faisceaux ultrasonores, obtenue selon le principe de l'invention ainsi que la forme de la zone focale de recouvrement des faisceaux ultrasonores, obtenue selon le principe de focalisation naturelle.

[Fig. 9D]La Figure 9D est une vue en perspective illustrant la forme de la zone focale de recouvrement désaxée des faisceaux ultrasonores, obtenue selon le principe de l'invention.

[Fig. 10A]La Figure 10A est une vue schématique montrant un autre exemple de réalisation d'une surface virtuelle de création d'un champ de pression d'ondes ultrasonores focalisées, obtenue par un transducteur réalisé selon la première variante de réalisation et tel qu'illustré à la Fig. 8A.

[Fig. 10B]La Figure 10B est une vue en coupe élévation dans le plan de profil illustrant le principe de focalisation par le transducteur illustré à la Fig. 8A pour réaliser une surface virtuelle de création d'un champ de pression d'ondes ultrasonores focalisées, montrée à la Fig. 10A de manière à obtenir une zone focale de recouvrement désaxée des faisceaux ultrasonores illustrée par rapport à la zone focale de recouvrement des faisceaux ultrasonores, obtenue selon le principe de focalisation naturelle.

[Fig. 10C]La Figure 10C est une vue en coupe prise perpendiculairement à l'axe de symétrie selon un plan Pr illustrant la forme de la zone focale de recouvrement désaxée des faisceaux ultrasonores, obtenue selon le principe de l'invention ainsi que la forme de la zone focale de recouvrement des faisceaux ultrasonores, obtenue selon le principe de focalisation naturelle.

[Fig. 10D]La Figure 10D est une vue en perspective illustrant la forme de la zone focale de recouvrement désaxée des faisceaux ultrasonores, obtenue selon le principe de focalisation illustré à la Fig. 10B.

[Fig. 10E]La Figure 10E est une vue en perspective illustrant la forme combinée des zones focales de recouvrement désaxées des faisceaux ultrasonores, obtenue pour deux phases d'exposition ultrasonores successives correspondant aux principes illustrés aux Fig. 9B et 10B.

[Fig. 10F]La Figure 10F est une vue de dessus illustrant la forme combinée des zones focales de recouvrement désaxées des faisceaux ultrasonores, obtenue pour deux phases d'exposition ultrasonores successives correspondant aux principes illustrés aux Fig. 9B et 10B.

[Fig. 11A]La Figure 11A est une vue de dessus illustrant lors de phases d'exposition successives, des zones focales de recouvrement désaxées concentriques et asymétriques.

[Fig. 11B]La Figure 11B est une vue en perspective illustrant lors de phases d'exposition successives, des zones focales de recouvrement désaxées concentriques, symétriques et superposées.

[Fig. 11C]La Figure 11C est une vue en perspective illustrant lors de phases d'exposition successives, des zones focales de recouvrement désaxées concentriques, symétriques et superposées.

[Fig. 11D]La Figure 11D est une vue en perspective illustrant lors de phases d'exposition successives, des zones focales de de recouvrement naturelles centrées combinées à des zones focales de recouvrement désaxées concentriques, symétriques et superposées.

[Fig. 11E]La Figure 11E est une vue de dessus illustrant lors de phases d'exposition successives, une zone focale de recouvrement naturelle entourée par un anneau de focalisation à deux zones focales de recouvrement désaxées concentriques, lui-même entouré par un anneau de focalisation à quatre zones focales de recouvrement désaxées concentriques.

[Fig. 12]La Figure 12 est une vue en perspective illustrant un exemple d'un transducteur selon une deuxième variante de réalisation avec une forme pseudo-cylindrique permettant la mise en œuvre de l'invention.

[Fig. 12A]La Figure 12A est une vue en perspective illustrant lors d'une phase d'exposition, la forme de la zone focale de recouvrement des faisceaux ultrasonores obtenue selon le principe de focalisation naturelle centrée réalisée par le transducteur avec une forme pseudo-cylindrique illustré à la Fig. 12.

[Fig. 12B]La Figure 12B est une vue en perspective illustrant lors d'une phase d'exposition, une forme de la zone focale de recouvrement désaxée des faisceaux ultrasonores, obtenue par le transducteur avec une forme pseudo-cylindrique illustré à la Fig. 12.

[Fig. 12C]La Figure 12C est une vue en perspective illustrant, lors d'une phase d'exposition subséquente, une forme symétrique de la zone focale de recouvrement désaxée des faisceaux ultrasonores, obtenue par le transducteur avec une forme pseudo-cylindrique illustré à la Fig. 12.

[Fig. 13A]La Figure 13A est une vue en perspective illustrant un exemple de mise en œuvre de la deuxième variante de réalisation du transducteur avec une forme pseudo-cylindrique illustré à la Fig. 12.

[Fig. 13B]La Figure 13B est une vue en perspective illustrant lors de deux phases d'exposition successives, la forme symétrique de la zone focale de recouvrement désaxée des faisceaux ultrasonores, obtenue par le transducteur illustré à la Fig. 13A.

[Fig. 13C]La Figure 13C est une vue en perspective illustrant lors de phases d'exposition successives, la forme des zones focales de recouvrement désaxées des faisceaux ultrasonores, obtenue par le transducteur illustré à la Fig. 13A.

[Fig. 13D]La Figure 13D est une vue en perspective illustrant lors de phases d'exposition successives, la forme des zones focales de recouvrement désaxées des faisceaux ultrasonores, obtenue par le transducteur illustré à la Fig. 13A, en relation avec une zone à préserver des ondes ultrasonores.

Description des modes de réalisation

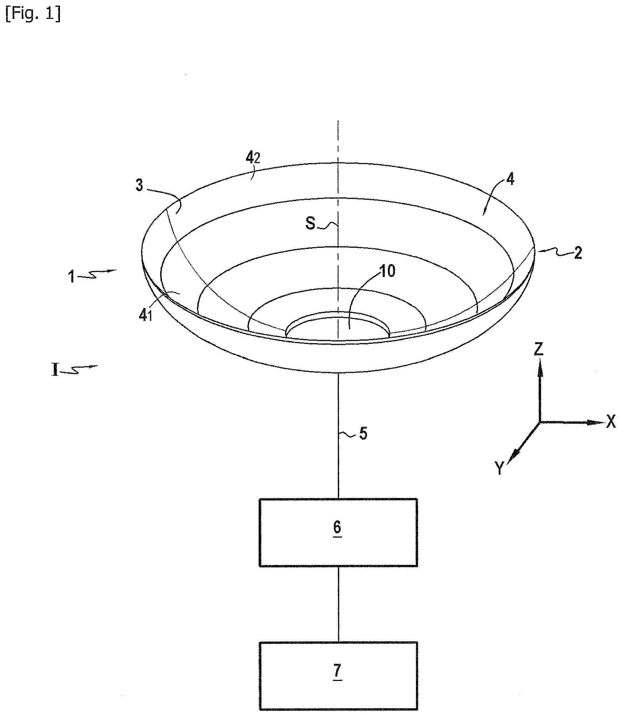

Tel que cela ressort plus précisément de la Fig. 1, l'objet de l'invention concerne un appareil de thérapie I au sens général comportant une sonde de thérapie 1 adaptée pour réaliser le traitement de tissus d'un être vivant par l'intermédiaire d'ultrasons focalisés de haute intensité (HIFU). La sonde de thérapie 1 comporte notamment un transducteur 2 comportant plusieurs émetteurs ultrasonores 3 tels que par exemple des éléments piézoélectriques, définissant ainsi une face 4 d'émission d'ondes ultrasonores focalisées. Ces émetteurs ultrasonores 3 sont reliés par l'intermédiaire de câbles coaxiaux 5 via, un étage amplificateur 6 à un circuit de commande 7 délivrant des signaux pour activer les émetteurs ultrasonores 3. Le circuit de commande 7 n'est pas décrit plus précisément car sa réalisation fait partie des connaissances techniques de l'homme du métier. Ce circuit de commande 7 comporte ainsi classiquement un générateur de signal piloté qui est relié aux émetteurs ultrasonores par l'intermédiaire de l'étage amplificateur 6. Ainsi, chaque émetteur ultrasonore 3 est relié à son propre générateur de signal.

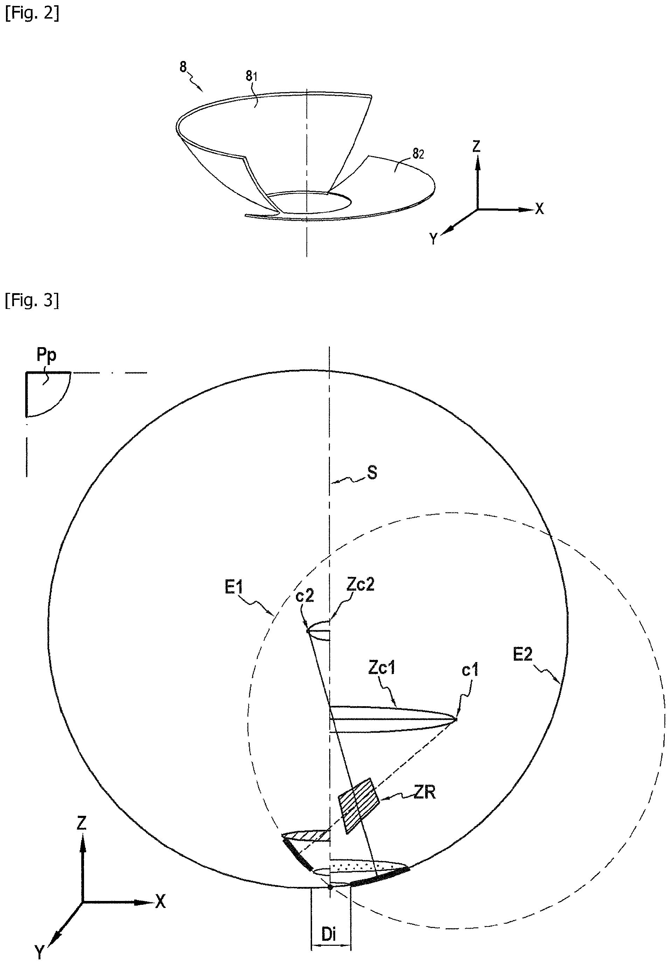

Le générateur de signal du circuit de commande 7 active les émetteurs ultrasonores 3 répartis en segments et plus précisément en segments de courbes ou de droites pour définir une surface 8 de création d'un champ de pression d'ondes ultrasonores focalisées. Selon un premier mode avantageux de réalisation, le générateur de signal faisant partie du circuit de commande 7 est piloté pour délivrer des signaux pour activer les émetteurs ultrasonores 3 du transducteur 2, avec une loi de retards ou de phases afin de réaliser la surface de création 8 d'un champ de pression d'ondes ultrasonores focalisées, cette surface de création 8 étant considérée comme une surface de création virtuelle distincte de la face 4 d'émission du transducteur (Fig. 2, 9A, 10A). Selon un deuxième mode de réalisation, la surface de création 8 d'un champ de pression d'ondes ultrasonores focalisées correspond à la face 4 du transducteur.

En d'autres termes, la surface de création 8 d'un champ de pression d'ondes ultrasonores focalisées correspond soit au transducteur physique ou plus précisément à la face 4 du transducteur 2 soit à une surface de création virtuelle 8 en appliquant des phases sur les voies de commande du transducteur 2.

Même si l'activation des émetteurs ultrasonores 3 par une loi de retards ou de phases est bien connue de l'homme du métier, la description qui suit en rappelle les principes.

P(r) la pression totale déposée en chaque point de la zone d'intérêt est définie par (Chavrier et coll. 2000) :

$$P(\vec{r}) = \frac{j.\rho.c}{\lambda} . \sum_{n=1}^{N} \int_{S} u_n . e^{j.\varphi_n} . \frac{e^{-(f.\alpha_{milieu}+j.k).\vec{r}}}{\vec{r}} . dS$$

Avec P(r) la pression (Pa) au point r, dS un ensemble de sources élémentaires, p la masse volumique du milieu de propagation, c la vitesse des ondes ultrasonores dans le milieu de propagation, $\lambda$ la longueur d'onde, N le nombre d'éléments actifs du transducteur, S la surface de chaque source d'émission, $u_n$ la vitesse normale de l'élément n, $\varphi_n$ la phase appliquée à l'élément n, $\alpha_{milieu}$ le coefficient d'atténuation du milieu, f la fréquence d'utilisation du transducteur et enfin k le nombre d'onde. Selon la méthode dite de la « réponse maximale » (Curiel et coll. 2002), la phase issue de chaque élément du transducteur au point de focalisation désiré est obtenu en fixant $\varphi_n$ = 0 dans l'intégrale linéaire citée au-dessus.

Ainsi la phase appliquée à chaque élément pour obtenir un signal maximal au point souhaité est donnée par :

$$Phase\,[(n, M)] = arctan\,\frac{Im[P(n, m)]}{Re[P(n, m)]}$$

Avec $Im[P(n,m)]$ et $Re[P(n,m)]$ respectivement les parties imaginaires et réelles du champ de pression au point M émis par l'élément n du transducteur. Ainsi pour chaque élément, n, le retard $\tau_n$ est défini par :

$$\tau_n = \frac{Phase[P(n,M)]}{2\pi f}$$

Une autre méthode de calcul consiste elle à établir la différence de chemin, pour chaque élément n, émettant entre le point de focalisation naturel et le point de focalisation désiré. Le retard de chaque élément est alors définit par $\tau_n = \frac{[d_{nat}]-[d_{foc}]}{c}$ avec $d_{nat}$ la distance entre l'élément n et son point de focalisation naturel, $d_{foc}$ la distance entre l'élément n et le point de focalisation désiré et c la vitesse des ondes ultrasonores dans le milieu de propagation. La phase est alors obtenue par $\varphi = \frac{\tau_n * 360}{1/c}$.

Une autre méthode de calcul consiste à définir un émetteur ultrasonore virtuel, de géométrie différente mais également découpé en N éléments puis de faire coïncider dans l'espace le centre du premier élément de l'émetteur réel avec le centre du premier élément de l'émetteur virtuel. La distance $d_n$ entre chaque élément respectif permet alors de calculer le retard à appliquer, définit par $\tau_n = \frac{[d_n]}{c}$ avec c la vitesse des ondes ultrasonores dans le milieu de propagation. La phase est alors obtenue par $\varphi = \frac{\tau_n * 360}{1/c}$.

Comme cela sera mieux compris dans la suite de la description, la forme de la surface de création 8 change notamment en fonction des applications de l'appareil de thérapie. Il apparaît donc avantageux de disposer d'une sonde avec une face 4 de forme déterminée facile à réaliser par l'homme du métier (Fig. 1 par exemple) et de générer à partir de cette face 4, des surfaces de création 8 qui ont des formes complexes à réaliser et différentes de la forme de la face 4 (comme illustré à la Fig. 2 par exemple). Cependant, il peut être envisagé de réaliser un transducteur 2 avec une face 4 dont la forme correspond à une surface de création 8 d'un champ de pression d'ondes ultrasonores focalisées, comme celle illustrée à la Fig. 2 par exemple.

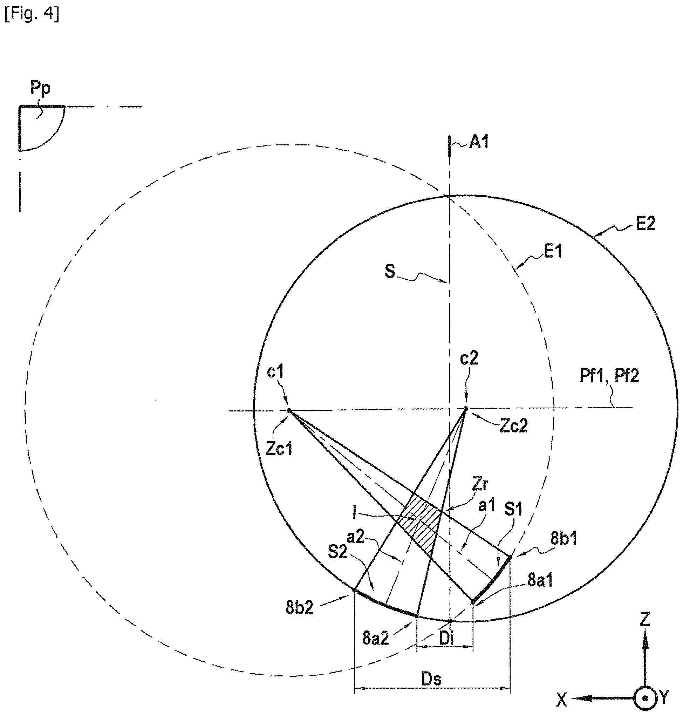

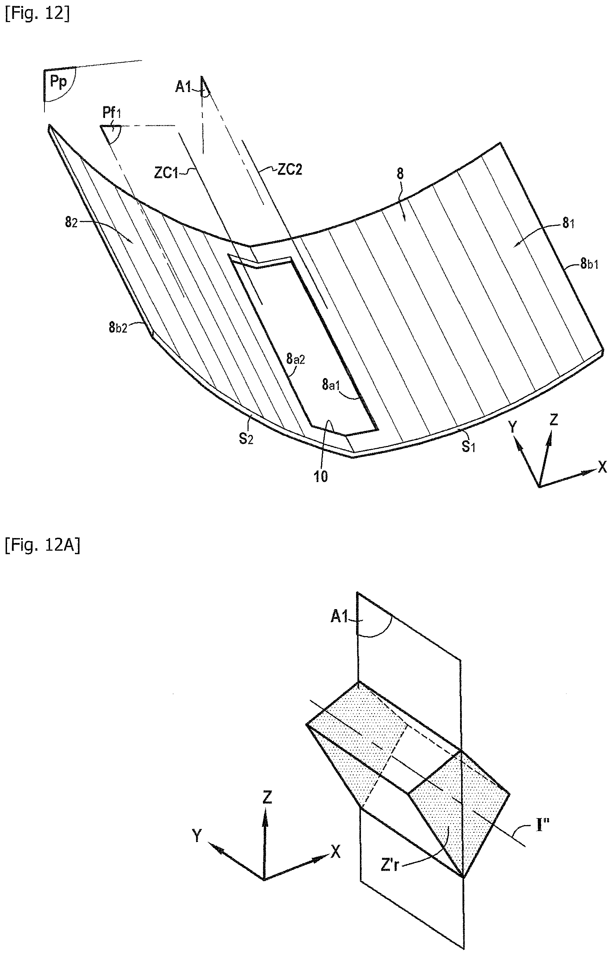

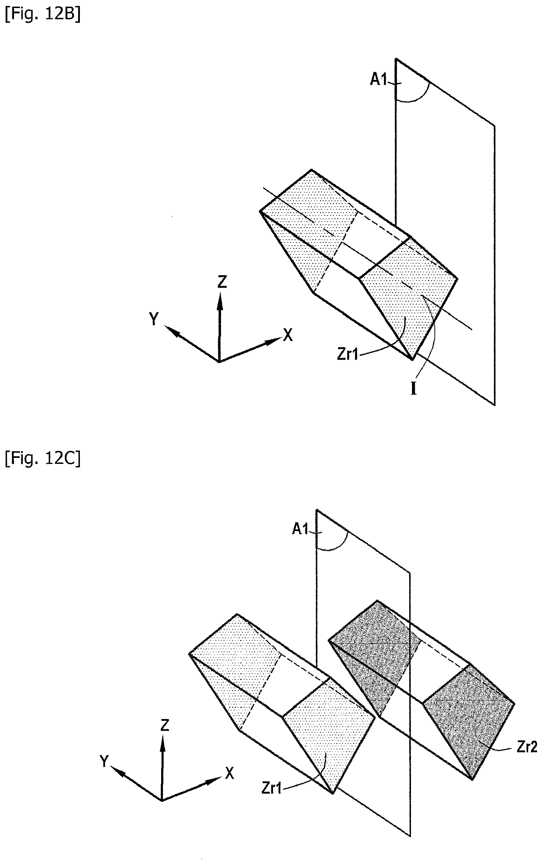

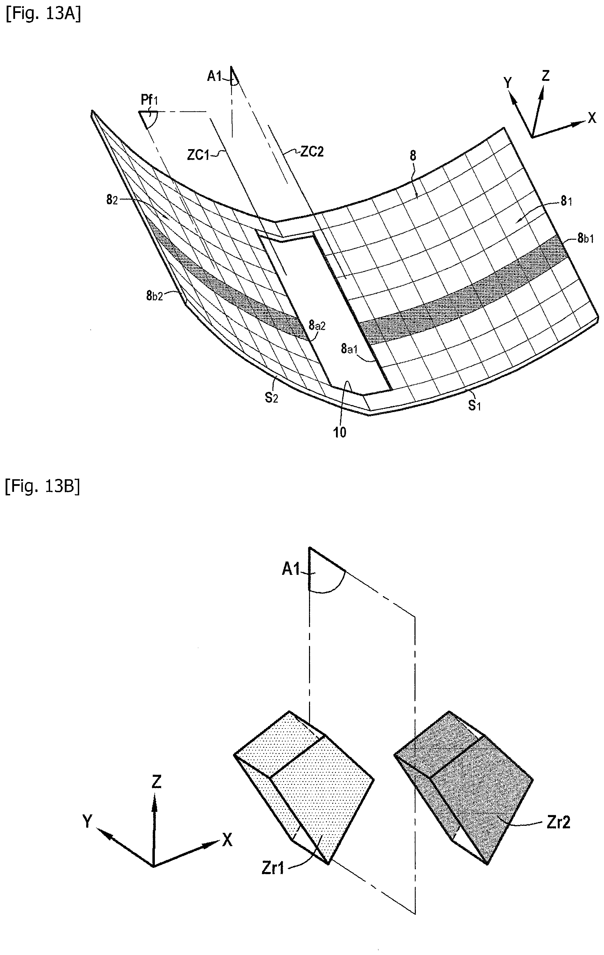

Il est à noter que la surface de création 8 présente selon une première variante de réalisation (Fig. 1, 2, 3, 7A-7C, 8A-8C, 9A-9D, 10A-10F, 11A à 11E), une forme de révolution autour d'un axe de symétrie S ou, selon une deuxième variante de réalisation (Fig. 12, 12A-12C, 13A-13D), une forme pseudo-cylindrique, tubulaire ou composée de deux portions de cylindre présentant un plan de symétrie A1. Il est à noter que les Fig. 4, 5, 6 et 6A sont des vues de profil permettant d'illustrer la surface de création 8 selon la première variante de réalisation et selon la deuxième variante de réalisation. L'axe de symétrie S qui s'établit selon la direction verticale Z correspond à l'axe de symétrie ou à l'axe acoustique de la face 4 du transducteur réalisée sous la forme d'une face de révolution. Le plan de symétrie A1 s'établit dans le plan défini par la direction verticale Z et la direction transversale Y, ce plan de symétrie étant perpendiculaire à un plan de profil Pp défini par les axes X, Z d'un repère X, Y, Z. Ce plan de symétrie A1 est le plan de symétrie ou le plan acoustique de la face 4 du transducteur.

Conformément à une caractéristique de l'invention illustrée plus particulièrement sur les Figures, la surface de création 8 est divisée en au moins N secteurs $8_1$, $8_2$, ..., selon une découpe radiale à l'axe acoustique S et contenant l'axe acoustique S ou s'étendant en position miroir par rapport au plan acoustique A1. Selon la première variante de réalisation, les secteurs sont des secteurs radiaux par rapport à l'axe acoustique S comportant pour chacun d'eux, des émetteurs ultrasonores 3 répartis en anneaux alors que dans la deuxième variante de réalisation, les secteurs sont des secteurs en position miroir par rapport au plan acoustique A1 comportant des émetteurs ultrasonores 3 répartis en segments linéaires. Selon la première variante de réalisation, les N secteurs radiaux sont compris avantageusement entre 2 et 8 et de préférence égale à 2 (Fig. 2) alors que dans la deuxième variante de réalisation, les N secteurs perpendiculaires au plan acoustique A1 sont compris entre 2 et 32 (Fig. 12 et 13A). Ainsi, selon ces variantes avantageuses de réalisation, les émetteurs ultrasonores 3 du transducteur sont en nombre limité, permettant de réduire le coût d'un tel transducteur. Par exemple, dans la variante préférée à deux secteurs, chaque secteur peut comporter 32 émetteurs ultrasonores 3. Par souci de simplification, uniquement le terme secteur sera utilisé dans la suite de la description.

La surface de création 8 est divisée en N secteurs $8_1$, $8_2$, ..., pour focaliser les ondes ultrasonores sur des zones focales respectivement $Zc_1$, $Zc_2$, ... s'établissant respectivement dans des plans focaux $Pf_1$, $Pf_2$, ...,. Les Fig. 2 à 6, 9A, 10A, 12 illustrent un exemple préféré de réalisation dans lequel la surface de création 8 est divisée en deux secteurs $8_1$, $8_2$. La Fig. 1 montre un autre exemple de réalisation pour lequel la surface d'émission 4 est avantageusement divisée en deux secteurs $4_1$, $4_2$, permettant de créer une surface de création 8 divisée en deux secteurs symétriquement opposés.

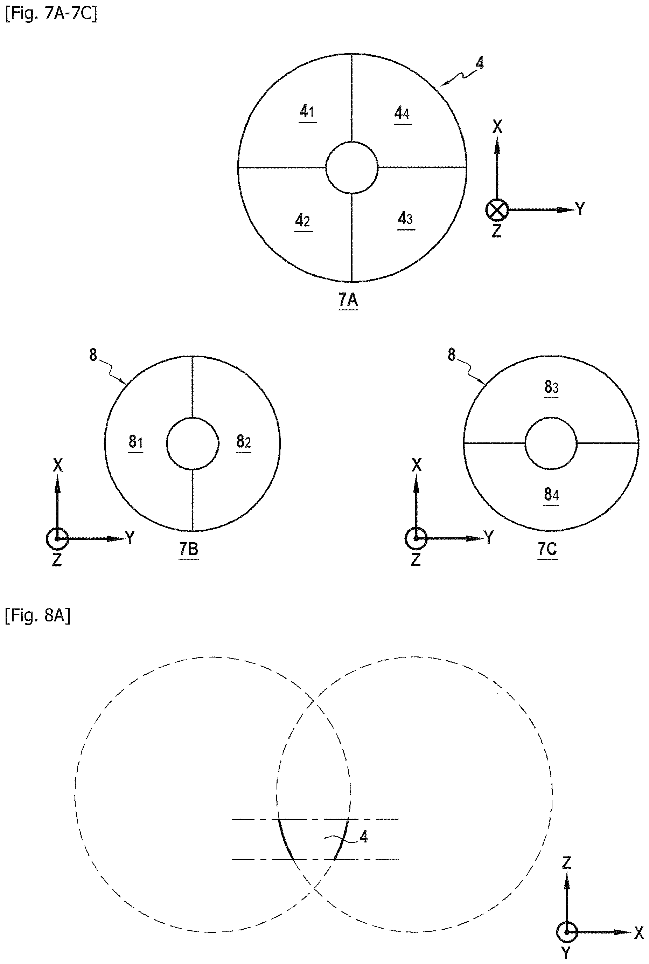

La Fig. 7A illustre un autre exemple de réalisation pour lequel la surface d'émission 4 est avantageusement divisée en quatre secteurs $4_1$, $4_2$, $4_3$, $4_4$, permettant ainsi de créer une surface de création 8 elle-même divisible jusqu'à quatre secteurs $8_1$, $8_2$, $8_3$, $8_4$. Avantageusement, la surface d'émission 4 est divisée en quatre secteurs afin de créer une surface de création 8 divisée en deux secteurs selon deux plans orthogonaux (Fig. 7B et 7C).

Tel que cela ressort plus précisément de la Fig. 4, chaque secteur $8_1$, $8_2$, ... de cette surface de création 8 présente dans un plan de profil Pp défini par les axes X, Z d'un repère X, Y, Z, un segment de courbe concave S1, S2, ... de longueur finie. Tel que cela ressort des Figures, les segments de courbe concave S1, S2 des secteurs $8_1$, $8_2$ sont délimités par des points extrêmes respectivement $8a_1$, $8b_1$ et $8a_2$, $8b_2$. Dans le plan profil Pp, les deux segments de courbe concave S1, S2, ... sont situés de part et d'autre du plan de symétrie A1 ou de l'axe de symétrie S.

Les segments de courbes concaves S1, S2, ... sont asymétriques par rapport à un axe de symétrie S du transducteur 2 comme dans la première variante de réalisation de la surface de création 8 avec une forme de révolution autour de l'axe de symétrie (Fig. 1, 2, 3, 7A-7C, 8A-8C, 9A-9D, 10A-10F, 11A à 11E) ou par rapport à un plan de symétrie A1 du transducteur selon la deuxième variante de réalisation de la surface de création 8 avec une forme pseudo-cylindrique (Fig. 12, 12A-12C, 13A-13D).

Conformément aux Fig. 4, 5, 6 et 6A qui illustrent la surface de création 8 selon la première variante de réalisation et selon la deuxième variante de réalisation, chaque segment de courbe concave S1, S2, ... possède un centre de courbure respectivement $C_1$, $C_2$, ... correspondant à une zone de focalisation ultrasonore Zc1, Zc2,... Conformément à l'invention, les segments de courbe concave S1, S2, ... des secteurs sont asymétriques par rapport au plan de symétrie A1 ou à l'axe de symétrie S. Aussi, les centres de courbures $c_1$, $c_2$, ... sont asymétriques par rapport au plan de symétrie A1 ou à l'axe de symétrie S dans la mesure où les centres de courbures $c_1$, $c_2$, ... sont situés à des distances différentes du plan de symétrie A1 ou de l'axe de symétrie S et/ou à des profondeurs différentes prises selon la direction verticale Z.

Selon l'exemple illustré à la Fig. 4, comportant un premier secteur $8_1$ et un deuxième secteur $8_2$, la distance du centre de courbure $c_1$ du premier secteur $8_1$ par rapport à l'axe de symétrie S ou le plan de symétrie A1 est plus grande que la distance du centre de courbure $c_2$ du deuxième secteur $8_2$ par rapport à l'axe de symétrie S ou le plan de symétrie A1, ces distances étant prises selon la direction X perpendiculaire à l'axe de symétrie S ou au plan de symétrie A1. Il est à noter que dans l'exemple illustré à la Fig. 4, les centres de courbure $c_1$, $c_2$ sont situés à une même profondeur selon la direction verticale Z (ou axe de symétrie S), c'est-à-dire qu'ils se trouvent situés selon une même droite perpendiculaire à l'axe de symétrie S ou au plan de symétrie A1. En d'autres termes, les centres de courbures $c_1$, $c_2$ sont situés selon des plans focaux respectivement $Pf_1$, $Pf_2$ qui sont confondus.

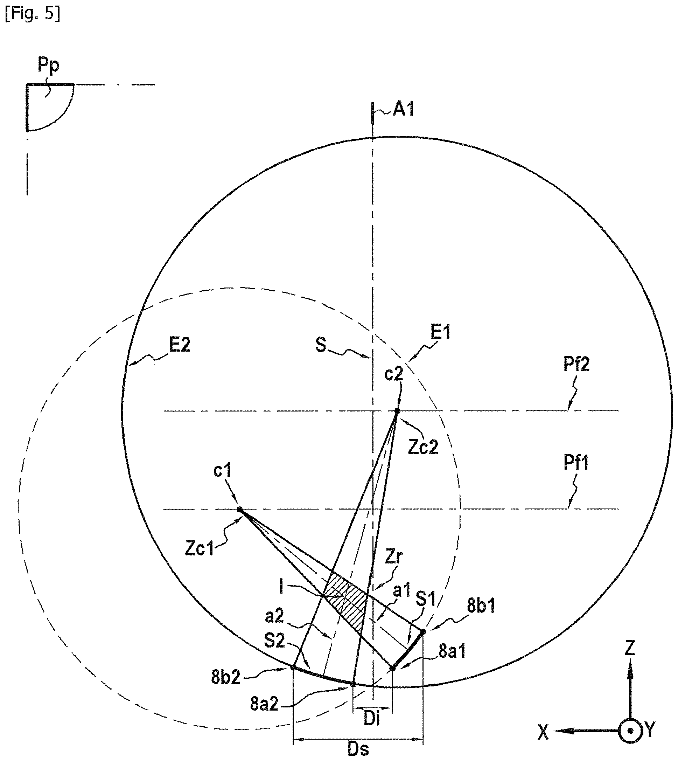

La Fig. 5 illustre un autre exemple de réalisation pour lequel la distance du centre de courbure $c_1$ par rapport à l'axe de symétrie S ou le plan de symétrie A1 est différente de la distance du centre de courbure $c_2$ par rapport à l'axe de symétrie S ou du plan de symétrie A1 mais également la profondeur selon l'axe de symétrie S, du centre de courbure $c_1$ est différente de la profondeur selon l'axe de symétrie S, du centre de courbure $c_2$. Ainsi, selon l'exemple illustré à la Fig. 5, le plan focal $Pf_2$ contenant le centre de courbure $c_2$ est selon l'axe de symétrie S, plus éloigné du transducteur que le plan focal $Pf_1$ contenant le centre de courbure $c_1$. En d'autres termes, les centres de courbures c1, c2 sont situés selon des plans focaux respectivement Pf1, Pf2 qui sont distincts.

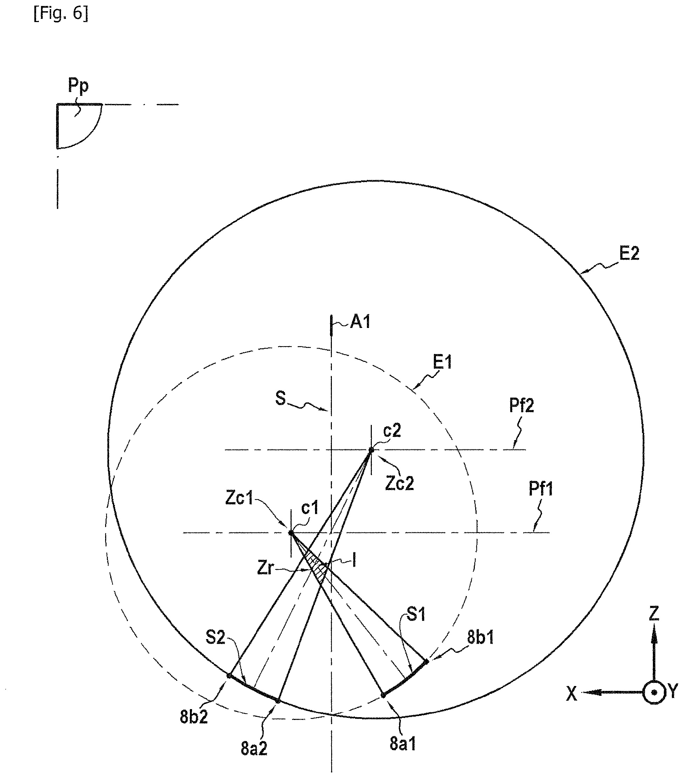

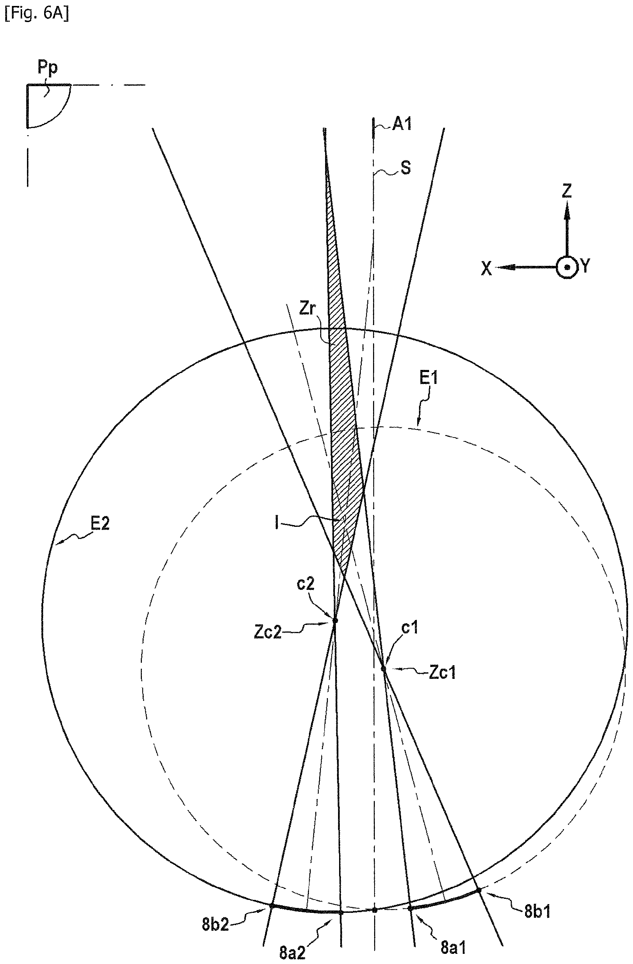

Bien entendu, les centres de courbures $c_1$, $c_2$ sont aussi considérés comme asymétriques dans le cas où les profondeurs selon l'axe de symétrie S sont différentes alors que les distances des centres de courbure $c_1$, $c_2$ par rapport

à l'axe de symétrie S ou au plan de symétrie A1 sont identiques. La Fig. 6 illustre cet exemple de réalisation pour lequel la distance du centre de courbure $c_1$ par rapport à l'axe de symétrie S ou le plan de symétrie $A_1$ est égale à la distance du centre de courbure $c_2$ par rapport à l'axe de symétrie S ou du plan de symétrie A1 alors que la profondeur selon l'axe de symétrie S, du centre de courbure $c_1$ est différente de la profondeur selon l'axe de symétrie S, du centre de courbure $c_2$. Selon cet exemple, les centres de courbures c1, c2 sont situés selon des plans focaux respectivement Pf1, Pf2 qui sont distincts.

Chaque segment de courbe concave S1, S2, ... des secteurs possède un axe propre respectivement $a_1$, $a_2$, ... passant par le centre de courbure $c_1$, $c_2$, ... dudit segment de courbe concave et le milieu dudit segment de courbe concave S1, S2, ....

Selon une autre caractéristique de l'invention, les axes propres $a_1$, $a_2$, ... des segments de courbe concave se coupent pour créer une zone focale de recouvrement Zr qui est désaxée par rapport au plan de symétrie A1 ou par rapport à l'axe de symétrie S. Cette zone focale de recouvrement Zr correspond à une zone focale de recouvrement des faisceaux ultrasonores provenant des secteurs $8_1$, $8_2$, ... de la surface de création 8. Dans les exemples de réalisation illustrés aux Fig. 4 à 6 et 6A, la zone focale de recouvrement Zr présente dans le plan de profil Pp, une section à quatre côtés en forme de parallélogramme, délimités par les faisceaux issus des points extrêmes $8a_1$, $8b_1$ et $8a_2$, $8b_2$ des segments de courbe concave respectivement S1, S2. Cette zone focale de recouvrement Zr des ondes ultrasonores est avantageusement utilisée pour créer une lésion biologique volumineuse. Indépendamment de l'exploitation de la zone focale de recouvrement Zr en tant que zone de traitement, la géométrie des lésions réalisées est maîtrisée par la combinaison des zones focales Zc1, Zc2, ... et de la zone focale de recouvrement Zr. Dans la présente demande, la zone de dépôt d'énergie correspond à la zone focale de recouvrement Zr, indépendamment de l'énergie éventuellement déposée dans les autres zones focales.

Cette zone focale de recouvrement Zr est désaxée dans le sens où les axes propres $a_1$, $a_2$, ... se coupent en un point d'intersection commun I qui est situé en dehors du plan de symétrie $A_1$ ou en dehors de l'axe de symétrie S. Les axes propres $a_1$, $a_2$, ... se coupent soit à une profondeur située entre les zones focales $Zc_1$, $Zc_2$, ... et la surface de création 8 comme illustré aux Fig. 3 à 6 et 12, soit au-delà des zones focales $Zc_1$, $Zc_2$, ... comme illustré à la Fig. 6A. Les faisceaux issus des secteurs $8_1$, $8_2$, ... se croisent de manière que la zone focale de recouvrement Zr se trouve située selon la direction verticale Z, à distance des plans focaux $Pf_1$, $Pf_2$, ....

Selon une caractéristique avantageuse de réalisation, les segments de courbe S1, S2, ... s'étendent dans le plan de profil Pp, de part et d'autre de l'axe de symétrie S ou du plan de symétrie A1, en étant disjoints pour permettre le positionnement de la zone focale de recouvrement Zr à distance de la surface de création 8. Ainsi, comme cela ressort clairement des Fig. 4 à 6 et 6A, les points extrêmes $8a_1$, $8a_2$ dits internes des segments de courbe sont écartés par rapport à l'axe de symétrie S ou au plan de symétrie A1.

Il ressort d'une telle disposition que la surface de création 8 et par suite, la face 4, présente une ouverture 10 centrée sur l'axe de symétrie S ou sur le plan de symétrie A1. Les points extrêmes dits internes $8a_1$, $8a_2$ des segments de courbe situés dans le plan de profil Pp sont écartés l'un de l'autre d'une distance interne Di prise selon l'axe X comprise entre 10 mm et 120 mm. Le choix de l'écart entre ces points extrêmes entraine une modification de la position de la zone focale de recouvrement Zr par rapport à la surface de création 8 (Fig. 3, 4, 5, 8B, 9B, 10B) ou une modification de la forme de la zone focale de recouvrement Zr et de son étalement selon l'axe Z (Fig. 6A) selon la configuration considérée. Avantageusement, cette ouverture 10 sert de logement pour une sonde d'imagerie ultrasonore.

Il est à noter que dans le plan de profil Pp, les points extrêmes dits externes $8b_1$, $8b_2$ des secteurs sont séparés d'une distance externe Ds permettant de localiser la zone focale de recouvrement Zr à distance des plans focaux. En d'autres termes, la zone focale de recouvrement Zr ne touche pas le plan focal $Pf_1$, $Pf_2$, .... Ainsi, la zone focale de recouvrement Zr et les zones focales $Zc_1$, $Zc_2$, ... sont distinctes ou séparées les unes des autres.

Selon une autre caractéristique de l'invention, les secteurs $8_1$, $8_2$, ... de la surface de création 8 sont engendrés soit, selon la première variante de réalisation, par la rotation de 2 $\Pi$/N des segments de courbe concave S1, S2, ... autour de l'axe de symétrie S avec N, le nombre de secteur, soit, selon la deuxième variante de réalisation, par la translation des segments de courbe S1, S2, ... selon une direction Y perpendiculaire au plan de profil Pp contenant lesdits segments de courbe S1, S2, .... Les secteurs $8_1$, $8_2$, ... s'étendent selon des plages angulaires ou des longueurs de valeurs sensiblement identiques. Ainsi, dans le cas où la surface de création 8 est une surface de révolution divisée en deux secteurs, alors chaque secteur $8_1$, $8_2$ s'étend selon une plage angulaire de 180° (Fig. 2).

Les secteurs $8_1$, $8_2$,..., situés en position miroir selon l'axe de symétrie S ou le plan de symétrie A1 créent des zones de dépôt d'énergie avec des profils correspondant aux zones focales de recouvrement Zr et des zones focales Zc. Selon la première variante de réalisation pour laquelle les secteurs de la surface de création 8 sont engendrés par la rotation des segments de courbe concave autour de l'axe de symétrie S, alors les zones focales $Zc_1$, $Zc_2$, ... présentent des formes en une portion de cercles. Dans le cas où la surface de création 8 est divisée en deux secteurs $8_1$, $8_2$, alors les zones focales $Zc_1$ et $Zc_2$ sont des demi-cercles s'étendant selon une plage angulaire de 180° (Fig. 3). Selon la deuxième variante de réalisation pour laquelle les secteurs $8_1$, $8_2$, ... sont engendrés par la translation des segments de courbe concave selon la direction Y perpendiculaire au plan de profil Pp contenant lesdits segments de courbe alors

les zones focales $Zc_1$, $Zc_2$, ... s'étendent selon des segments linéaires situés dans les plans focaux Pf1, Pf2, ..., parallèlement à la direction Y (Fig. 12). Dans le cas où la surface de création 8 est divisée en deux secteurs $8_1$, $8_2$, alors les zones focales $Zc_1$ et $Zc_2$ s'étendent selon deux segments linéaires situés dans les plans focaux Pf1, Pf2, parallèlement à la direction Y (Fig. 12).

La surface de création 8 d'un champ de pression d'ondes ultrasonores focalisées est obtenue ou réalisée à l'aide d'un transducteur dont la face 4 est adaptée pour obtenir les caractéristiques de la surface de création 8 décrites ci-dessus.

Selon la première variante de réalisation pour laquelle la surface de création 8 est de révolution alors la surface de création 8 est issue d'une face 4 définie par les éléments transducteurs, de géométrie avantageusement torique engendrée par la rotation de segments de courbe concave autour de l'axe de symétrie S de sorte que les segments de courbe concave suivent des arcs de cercles non coïncident qui se coupent de sorte que les zones focales $Zc_1$, $Zc_2$, ... possèdent une forme de portions de cercle.

Cette face 4 du transducteur correspond comme illustré à la Fig. 8A, à une découpe d'une portion de l'enveloppe d'un tore croisé, selon deux plans perpendiculaires à l'axe de symétrie S.

Selon la deuxième variante de réalisation pour laquelle la surface de création 8 présente une forme pseudo-cylindrique ou tubulaire, alors la surface de la surface de création 8 est issue d'une face 4 définie par les éléments transducteurs, de géométrie cylindrique engendrée par la translation selon une longueur limitée, de deux segments de courbe selon une direction Y perpendiculaire au plan de profil Pp contenant lesdits segments de courbe de sorte que les zones focales Zc1, Zc2 ... possèdent une forme linéaire.

La Fig. 8A donne un exemple de réalisation préféré de la face 4 du transducteur 2 définie par des segments de courbe concave s'appuyant sur une géométrie torique croisée.

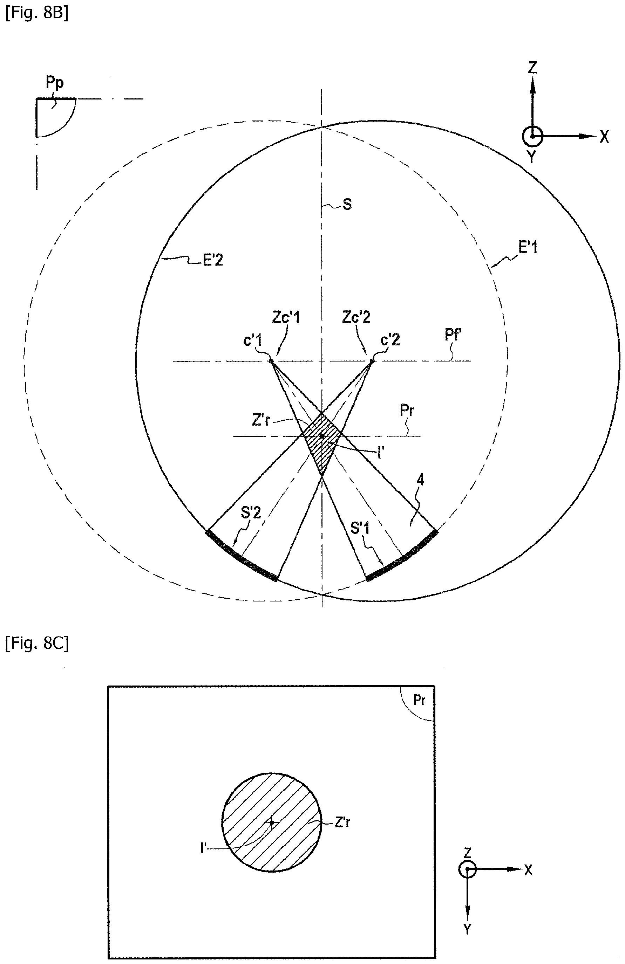

Tel que cela ressort de la Fig. 8B, la face d'émission 4 comporte dans le plan de profil Pp, deux segments de courbes concaves $S'_1$, $S'_2$ qui suivent les arcs respectivement d'un premier cercle E'1 possédant un centre $c'_1$ et d'un deuxième cercle E'$_2$ possédant un autre centre $c'_2$ différent du centre $c'_1$ du premier cercle E'1. Les premier et deuxième cercles E'1, E'2 ne coïncident pas mais se coupent entre eux. L'un des deux segments de courbe concave $S'_1$ (à droite sur la Fig. 8B), suit l'arc du premier cercle E'1, cet arc du premier cercle E'1 étant situé à l'intérieur du deuxième cercle E'2. De manière similaire, l'autre segment concave $S'_2$ (à gauche sur la Fig. 8B) suit l'arc du deuxième cercle E'2, cet arc du deuxième cercle E'2 étant situé à l'intérieur du premier cercle E'1.

Cette face d'émission 4 présente dans le plan de profil Pp, deux segments de courbe concave $S'_1$, $S'_2$ de longueur finie, symétrique par rapport à l'axe de symétrie S. Ainsi, chaque segment concave $S'_1$, $S'_2$ de la face d'émission 4 focalise les ondes ultrasonores au centre $c'_1$, $c'_2$ du cercle dans des zones de focalisation ultrasonore respectivement Zc'1, Zc'2 situées au-delà de l'axe de symétrie S par rapport à la face d'émission 4 correspondante $S'_1$, $S'_2$, ces zones de focalisation ultrasonore Zc'1, Zc'2 s'établissant dans un plan focal Pf'.

Les axes propres des segments de courbe concave $S'_1$, $S'_2$ coupent l'axe de symétrie S en un point d'intersection commun I' situé sur cet axe de symétrie S pour la première variante de réalisation. Ce point d'intersection commun des axes propres est situé entre la face d'émission 4 et les zones focales Zc'1, Zc'2 ou au-delà du plan focal Pf'. Ainsi, les faisceaux de la face d'émission 4 se croisent pour former la zone de recouvrement Z'r des faisceaux ultrasonores qui est symétrique par rapport à l'axe de symétrie S. Cette zone focale de recouvrement Z'r des faisceaux ultrasonores, dite naturelle est centrée sur l'axe de symétrie S.

Selon la première variante de réalisation pour laquelle le transducteur présente une forme de révolution, la face d'émission 4 est obtenue par la rotation autour de l'axe de symétrie S, d'un segment de courbe concave illustré par $S'_1$ et $S'_2$ en vue de coupe dont le centre de courbure est situé du côté opposé audit segment de courbe par rapport à l'axe de symétrie S. La Fig. 8C illustre dans le plan XY, la forme de la zone focale de recouvrement Z'r naturelle obtenue par un tel transducteur de révolution. Il est à rappeler que dans le cas d'un transducteur de forme torique, le transducteur dépose également de la pression dans un plan focal sous la forme d'un cercle représenté par les zones focales Zc'1, Zc'2 (Fig. 8B).

Selon cette première variante de réalisation, la face d'émission 4 est une surface de révolution. Cette face d'émission 4 comporte par exemple, une série d'éléments transducteurs ultrasonores 3 montés de manière concentrique les uns par rapport aux autres et par rapport à l'axe de symétrie S. Bien entendu, il peut être envisagé comme illustré à la Fig. 8A, que la face d'émission 4 soit tronquée de manière symétrique par rapport à l'axe de symétrie S afin que le transducteur se limite à une portion d'un tore croisé présentant une largeur comprise, par exemple, entre 5 et 250 mm. Selon cet exemple de réalisation, le transducteur 2 comporte une série d'éléments transducteurs ultrasonores 3 montés de manière concentrique les uns par rapport aux autres et par rapport à l'axe de symétrie S de sorte qu'ils sont répartis sous la forme de segments en forme d'anneau.

Selon la deuxième variante de réalisation pour laquelle le transducteur 2 présente une face d'émission 4 composée de deux portions de cylindre alors la face d'émission 4 est obtenue par la mise en oeuvre selon le principe décrit ci-dessus, de deux segments de courbe concave $S'_1$, $S'_2$ de longueur finie, symétrique par rapport au plan de symétrie A1 et la translation de ces deux segments de courbe concave $S'_1$, $S'_2$ selon la direction Y perpendiculaire au plan de profil Pp contenant lesdits segments de courbe concave. Ainsi, chaque partie de la face d'émission 4 focalise avant, après ou sur le plan de symétrie $A_1$, selon un segment linéaire s'étendant dans le plan focal, parallèlement à la direction Y. Les axes propres des segments de courbe concave $S'_1$, $S'_2$ coupent le plan de symétrie A1 en un axe d'intersection I" compris dans le plan de symétrie A1 (Fig. 12A). Comme expliqué précédemment pour la première variante de réalisation, lors d'une phase d'exposition naturelle, les faisceaux de la face d'émission 4 se croisent pour former la zone de recouvrement Z'r des faisceaux ultrasonores qui est symétrique par rapport au plan de symétrie A1. Cette zone focale de recouvrement Z'r des faisceaux ultrasonores, dite naturelle est centrée sur le plan de symétrie A1.

A l'aide de la sonde 1 pourvue de la face d'émission 4 décrite ci-dessus, est créée la surface de création 8 conforme à l'invention dont les caractéristiques ont été décrites précédemment. Dans la description qui suit, la surface de création 8 est divisée en deux secteurs $8_1$, $8_2$ à titre d'exemple préférée de réalisation mais l'objet de l'invention peut être mis en oeuvre pour une surface de création 8 comportant un nombre supérieur de secteurs. Il est à noter que le transducteur 2 correspond au transducteur physique ou réel tenu entre les mains de l'utilisateur et que la surface de création 8 est formée par deux demi-transducteurs virtuels simulés en appliquant des phases sur chacune des voies du transducteur physique après avoir subdivisé les deux secteurs en plusieurs éléments émetteurs. La détermination de ces phases consiste alors simplement à calculer, pour chaque élément émetteur, le temps de propagation des ultrasons entre le transducteur 2 et la surface de création 8. Selon l'invention, il est créé virtuellement deux parties de transducteurs permettant de déplacer les éléments de focalisation sans déplacer les éléments géométriques propres au transducteur physique comme l'axe acoustique ou l'axe de symétrie. Dans la description qui suit, il est décrit un mode de réalisation avantageux pour lequel le générateur de signal faisant partie du circuit de commande 7 est piloté pour délivrer des signaux pour activer les émetteurs ultrasonores 3 répartis en anneaux, avec une loi de retards ou de phases afin de réaliser la surface de création 8 d'un champ de pression d'ondes ultrasonores focalisées.

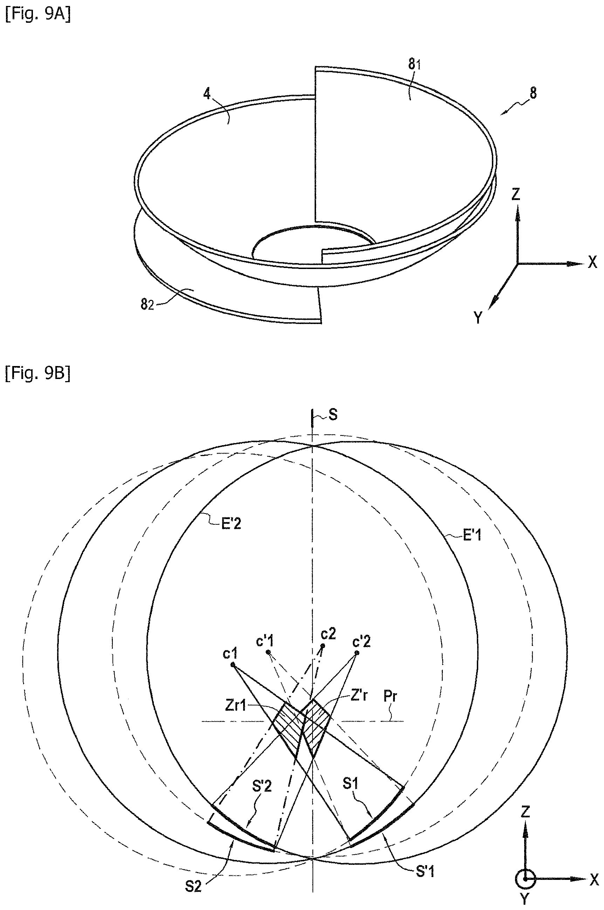

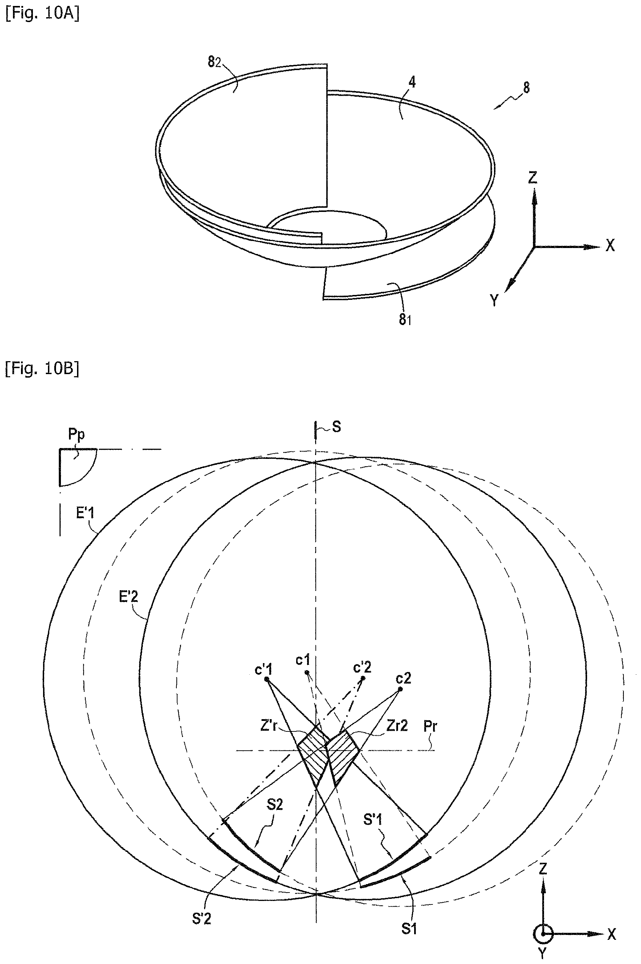

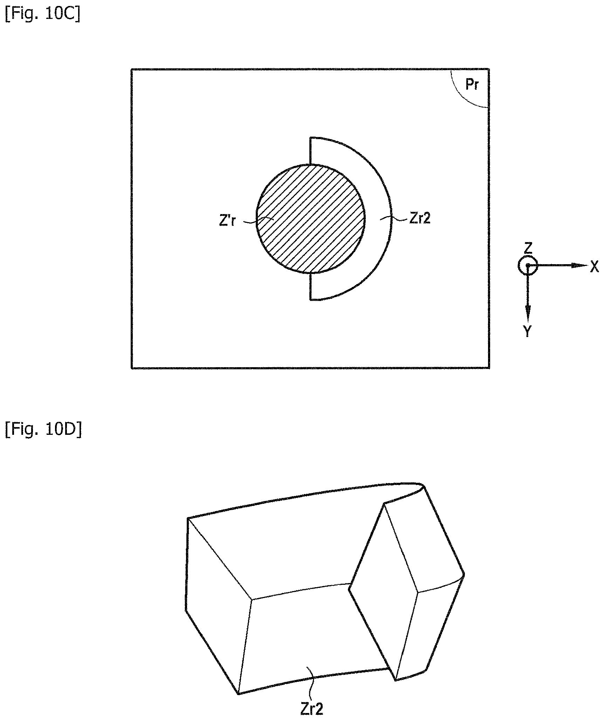

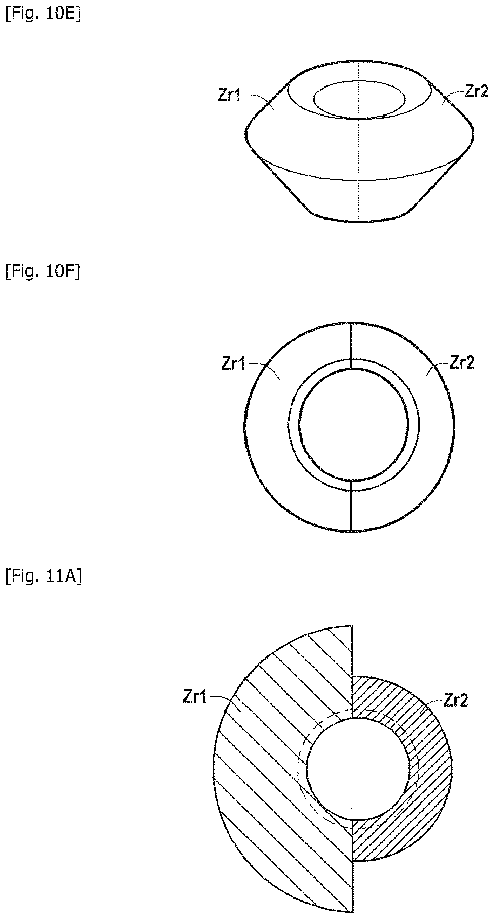

A titre d'exemple, les Fig. 9A - 9D et 10A - 10F illustrent le fonctionnement des émetteurs ultrasonores 3 du transducteur illustré aux Fig. 8A - 8C, pour réaliser la surface de création 8 du type de la première variante de réalisation comportant deux secteurs $8_1$, $8_2$.

Le générateur de signal faisant partie du circuit de commande 7 est piloté pour délivrer des signaux pour dans une phase d'exposition, activer les émetteurs ultrasonores 3 faisant partie d'un secteur $4_1$ et du secteur symétriquement opposé $4_2$ par rapport à l'axe de symétrie S, pour créer la zone de dépôt d'énergie correspondante.

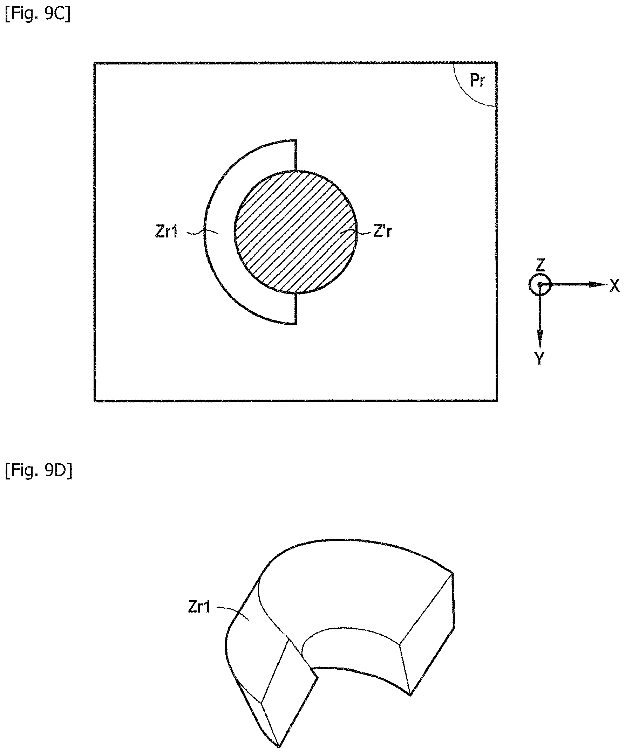

Dans une phase d'exposition, les émetteurs ultrasonores 3 des deux secteurs $4_1$, $4_2$ sont activés pour obtenir la surface de création 8 du champ de pression d'ondes ultrasonores focalisées de façon que cette dernière forme deux faces d'émission, chacune obtenue par la rotation autour de l'axe de symétrie S, de segments de courbe concave $s_1$ et $s_2$ différents. Ainsi les centres de courbure $c_1$, $c_2$ peuvent être situés indépendamment pour chaque secteur $8_1$, $8_2$ du côté opposé ou non audit segment de courbe par rapport à l'axe de symétrie, permettant ainsi de créer une zone de dépôt d'énergie Zr1 désaxée par rapport à l'axe de révolution, comme illustré sur les Fig. 9B et 9C. L'activation des émetteurs ultrasonores 3 faisant partie du secteur $8_1$ et du secteur $8_2$ symétriquement opposé permet de créer une zone de dépôt d'énergie et notamment une zone arquée de dépôt d'énergie $Zr_1$ avec un profil correspondant à la zone focale de recouvrement. Ce principe s'applique pour l'ensemble des secteurs composant le transducteur, permettant d'affiner la forme de la zone dans laquelle est déposée la pression.

Dans une deuxième phase d'exposition illustrée plus précisément aux Fig. 10A - 10D, les émetteurs ultrasonores 3 des deux secteurs $4_1$, $4_2$ sont activés pour créer par rapport à la zone arquée de dépôt d'énergie $Zr_1$ illustrée à la Fig. 9D, une zone arquée de dépôt d'énergie $Zr_2$, symétrique avec un profil correspondant à la zone focale de recouvrement. Pour réaliser cette deuxième phase d'exposition, le circuit de commande va de nouveau activer la totalité de la surface du transducteur en inversant les phases appliquées entre les secteurs par rapport au principe décrit précédemment.

Il doit être compris que le générateur de signal faisant partie du circuit de commande 7 est piloté pour délivrer des signaux pour, dans des phases d'exposition successives, activer les émetteurs ultrasonores faisant partie de chacun des secteurs et de chaque secteur symétriquement opposé par rapport à l'axe de symétrie, de manière à créer, pour chaque paire de secteurs, la zone de dépôt d'énergie correspondante. Dans l'exemple illustré (Fig. 10E - 10F), il est possible par deux expositions ultrasonores successives, de créer une couronne de coagulation.

Dans l'exemple de réalisation illustrée aux Fig. 10E - 10F, la couronne de coagulation présente un profil ou une largeur constante. Bien entendu, il est possible de faire varier d'une phase d'exposition à l'autre, le profil ou la largeur des zones arquées de dépôt d'énergie, ainsi que la hauteur selon la direction Z tout comme leurs positions par rapport à l'axe de symétrie S.

Ainsi, d'une manière générale, le générateur de signal faisant partie du circuit de commande 7 est piloté pour délivrer des signaux pour activer les émetteurs ultrasonores dans des phases d'exposition successives pour chacune desquelles les centres de courbure $c_1$, $c_2$, ... sont situés à des distantes différentes du plan de symétrie $A_1$ ou de l'axe de symétrie S et/ou à des profondeurs différentes selon l'axe de vertical Z de manière à obtenir des zones de dépôt d'énergie désaxées.

Ainsi, le générateur de signal faisant partie du circuit de commande 7 est piloté pour délivrer des signaux pour activer les émetteurs ultrasonores dans des phases d'exposition successives pour lesquelles les centres de courbure

$c_1$, $c_2$, ... sont situés à des distances différentes du plan de symétrie A1 ou de l'axe de symétrie S et/ou à des profondeurs différentes selon l'axe vertical Z de manière à obtenir pour ces phases d'exposition successives, des zones de dépôt d'énergie désaxées, de positions différentes avec des tailles identiques ou différentes.

Dans l'exemple illustré aux Fig. 10E - 10F, les émetteurs ultrasonores de l'ensemble des deux secteurs $8_1$, $8_2$ ont subi deux phases d'activation pour lesquelles les phases ont été calculées de façon à ce que les zones focales de recouvrement Zr1 et Zr2 soient symétriques par rapport à l'axe de symétrie S. Cela se traduit au niveau de la surface de création 8 par la transformation du segment de courbe concave $S_1$ en $S_2$ et du segment de courbe concave $S_2$ en $S_1$ lors du second cycle d'activation.

La Fig. 11A illustre un exemple de réalisation dans lequel pour un cycle d'exposition les émetteurs ultrasonores de l'ensemble des deux secteurs $8_1$, $8_2$ ont subi deux phases d'activation pour lesquelles les phases ont été calculées de façon à ce que les zones focales de recouvrement Zr1 et Zr2 soient asymétriques par rapport à l'axe de symétrie S. Selon cet exemple, les phases d'activations sont réalisées avec des centres de courbure $c_1$, $c_2$, positionnés de manière adaptée pour obtenir des zones focales de recouvrement Zr1 et Zr2 asymétriques.

Il apparaît ainsi possible de réaliser successivement les phases d'exposition, de manière que d'une phase d'exposition à l'autre, les centres de courbure soient situés distinctement :

- soit à des distances différentes par rapport au plan de symétrie A1 ou l'axe de symétrie S mais à des profondeurs identiques ;
- soit à une même distance selon le plan de symétrie A1 ou l'axe de symétrie S mais à des profondeurs différentes ;
- soit à des distances différentes par rapport au plan de symétrie A1 ou l'axe de symétrie S et à des profondeurs différentes selon le plan de symétrie A1 ou l'axe de symétrie S.

Il apparaît ainsi possible pour, dans des phases d'exposition successives, activer les émetteurs ultrasonores de manière que les distances et/ou les profondeurs des centres de courbure sont modifiées d'une phase d'exposition à l'autre afin que les zones focales de recouvrement soient concentriques et/ou symétriques et/ou asymétriques et/ou superposées selon l'axe vertical Z. Les phases d'exposition peuvent ainsi être combinées à « l'infini » en fonction de la forme plus ou moins complexe du volume à traiter.

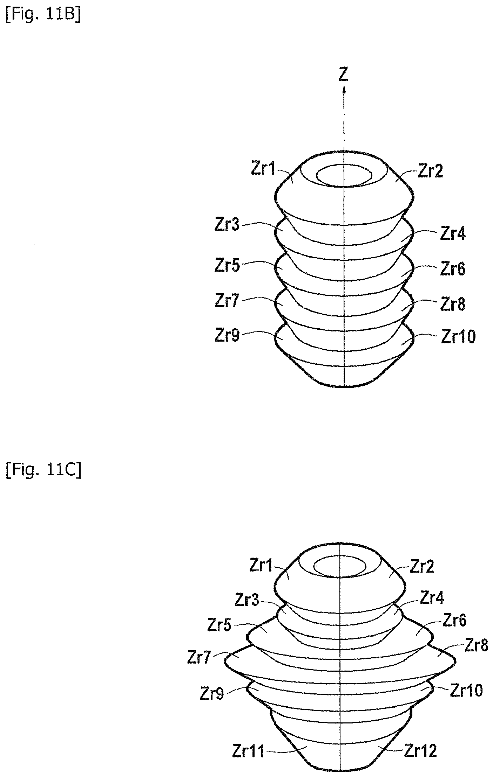

La Fig. 11B illustre un exemple de réalisation pour lequel le générateur de signal faisant partie du circuit de commande 7 est piloté de manière que les émetteurs ultrasonores de l'ensemble des deux secteurs $8_1$, $8_2$ subissent deux phases d'activation pour lesquelles les phases ont été calculées de façon à ce que les zones focales de recouvrement Zr1 et Zr2 soient symétriques par rapport à l'axe de symétrie S. Ces deux phases d'activation forment un cycle d'exposition qui est répété pour des profondeurs différentes selon l'axe vertical Z afin d'obtenir des couronnes superposés de dépôt d'énergie formant un cylindre présentant une largeur et une hauteur identiques d'un cycle d'exposition à l'autre.

La Fig. 11C illustre un autre exemple de réalisation pour lequel le générateur de signal faisant partie du circuit de commande 7 est piloté pour délivrer des signaux pour activer les émetteurs ultrasonores 3 de manière que les zones focales de recouvrement Zr soient situés à des distances différentes et à des profondeurs différentes selon l'axe vertical Z de manière à obtenir des zones focales de recouvrement superposées de largeurs et/ou de hauteurs variables.

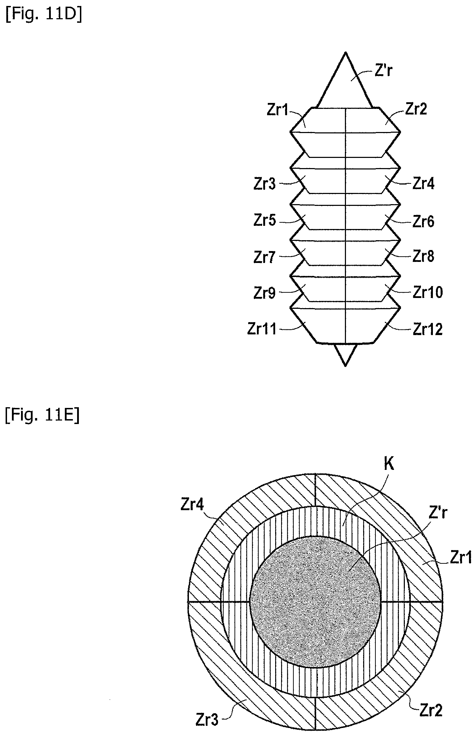

Dans les exemples décrits qui précèdent, le volume de dépôt d'énergie est creux c'est-à-dire pas en contact avec l'axe de symétrie S. Bien entendu, le générateur de signal faisant partie du circuit de commande peut être piloté pour, dans au moins une phase d'exposition complémentaire, délivrer des signaux pour activer les émetteurs ultrasonores de manière à assurer la focalisation des ondes ultrasonores dans des zones focales et à obtenir une zone focale de recouvrement Z'r centrée par rapport au plan de symétrie A1 ou à l'axe de symétrie S et située à distance des plans focaux entre les zones focales et la face d'émission ou au-delà des zones focales. Ainsi, il peut être réalisé une phase d'exposition complémentaire dite naturelle selon le principe décrit aux Fig. 8A et 8B, pour une ou chaque phase d'exposition de manière à venir compléter le volume de dépôt d'énergie. Bien entendu, cette phase d'exposition complémentaire peut être mise en œuvre pour l'un et/ou l'autre des exemples de réalisation décrits ci-avant. Ainsi, la Fig. 11D illustre la mise en œuvre d'une phase d'exposition complémentaire pour la variante illustrée à la Fig. 11B.

La Fig. 11E illustre en vue de dessus, une zone focale de recouvrement naturelle Z'r centrée par rapport à l'axe de symétrie S entourée par une couronne de focalisation K réalisée en deux expositions ultrasonores successives comme expliqué précédemment. Il est à noter que cette Figure illustre la réalisation autour de la couronne de focalisation K, d'une couronne supplémentaire de focalisation réalisée en quatre expositions ultrasonores successives mise en œuvre par une surface de création 8 divisée en quatre secteurs, pour créer quatre zones focales de recouvrement Zr1 à Zr4.

La description qui suit illustre à titre d'exemple, la mise en œuvre de la surface de création 8 relevant de la deuxième variante de réalisation. Selon cet exemple illustré à la Fig. 12, la première zone focale possède une forme linéaire double. Les conditions de mise en œuvre de l'invention décrites en relation des Fig. 10A-10F, 11A-11E pour la première variante de réalisation s'appliquent de manière analogue pour cette surface de création 8 de forme pseudo-cylindrique.

Conformément à la présente invention, les émetteurs ultrasonores 3 sont découpés parallèlement au plan de symétrie A1 et sont activés avec une loi de retards ou de phases afin de réaliser la surface de création 8. Les émetteurs ultrasonores sont répartis selon au moins deux secteurs et selon l'exemple illustré à la Fig. 12, deux secteurs $8_1$, $8_2$ disposés symétriquement par rapport au plan de symétrie A1.

Ainsi, le générateur de signal faisant partie du circuit de commande est piloté pour délivrer des signaux pour dans une phase d'exposition, activer les émetteurs ultrasonores faisant partie d'un secteur situé d'un côté du plan de symétrie A1 et du secteur symétriquement opposé pour créer la zone de dépôt d'énergie correspondante. Il est ainsi possible de réaliser un volume de dépôt d'énergie Zr1 (ou une zone focale de recouvrement Zr1) d'un côté du plan de symétrie A1 et à distance de ce plan de symétrie (Fig. 12B). De même, il est possible de réaliser un volume de dépôt d'énergie Zr2 (ou une zone focale de recouvrement Zr2) de l'autre côté du plan de symétrie A1 et à distance de ce plan de symétrie A1 (Fig. 12C).

Selon un autre exemple avantageux de réalisation, les émetteurs ultrasonores sont répartis selon plusieurs secteurs perpendiculaires au plan de symétrie A1 et disposés en vis-à-vis selon le plan de symétrie A1. Dans l'exemple illustré à la Fig. 13A, neuf secteurs sont situés de chaque côté du plan de symétrie A1, avec chaque secteur d'un côté étant en regard avec un secteur de l'autre côté. Ainsi, le générateur de signal faisant partie du circuit de commande est piloté pour délivrer des signaux pour dans une phase d'exposition, activer les émetteurs ultrasonores faisant partie d'un secteur et du secteur opposé, avec une loi de retards ou de phases pour créer la zone de dépôt d'énergie correspondante d'un côté du plan de symétrie (Fig. 13A). Les émetteurs ultrasonores des secteurs en vis-à-vis sont capables de créer des volumes de dépôt d'énergie de part et d'autre du plan de symétrie A1. La Fig. 13B illustre la réalisation de deux volumes de dépôt d'énergie situés de part et d'autre du plan de symétrie A1. L'épaisseur du volume de dépôt d'énergie correspond à la largeur d'un secteur du transducteur.

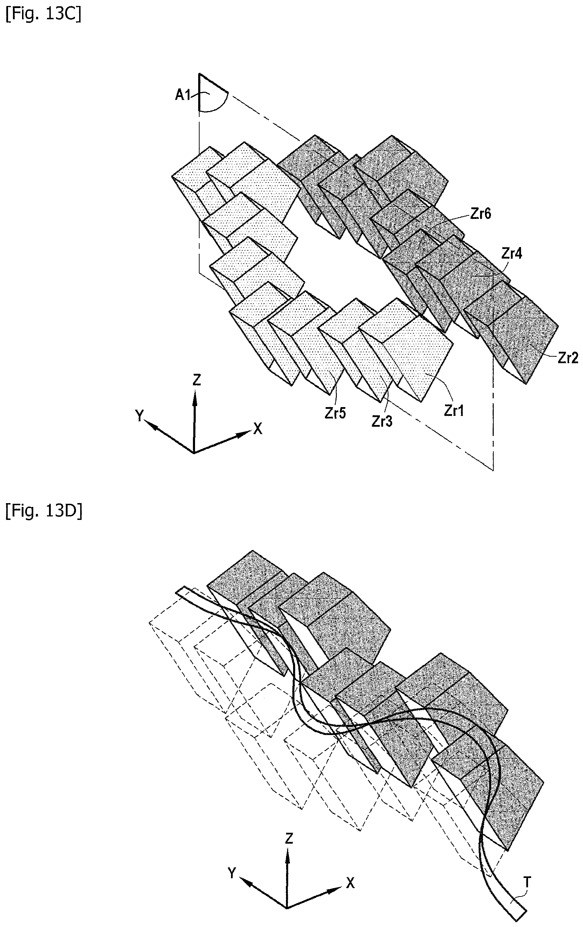

Comme expliqué précédemment, chaque volume de dépôt d'énergie peut varier en forme, en taille et en localisation en jouant notamment sur la position des centres de courbure. L'activation successive des différents secteurs du transducteur permet de réaliser un volume de dépôt d'énergie creux d'épaisseur variable (Fig. 13C).

Cette solution trouve des applications particulièrement avantageuse pour traiter des zones cibles s'étendant le long d'un segment non rectiligne T permettant par exemple le traitement de tumeurs le long des artères ou veines, le long du tube digestif, le long des os, le long du canal du Wirsung dans le pancréas, l'urètre ou le canal biliaire (Fig. 13D).

Il ressort de la description qui précède que l'objet de l'invention permet de réaliser un déplacement de la zone focale en profondeur (selon l'axe acoustique et un déplacement dans un plan parallèle au transducteur (perpendiculaire à l'axe acoustique) tout en réduisant le nombre d'émetteurs. Contrairement à l'art antérieur qui propose de créer un point focal et de le déplacer dans l'espace (focalisation électronique), l'objet de l'invention vise à créer directement un volume (portion de couronne) obtenu en calculant une surface de création « virtuelle » ou transducteur virtuel et à déplacer ce volume sans avoir recours à un nombre important d'émetteurs. Cette invention permet ainsi de réduire le coût de l'électronique de commande mais également le coût du transducteur.

## Revendications

1. Appareil de thérapie pour le traitement de tissus par l'émission d'ondes ultrasonores focalisées, comportant une sonde de thérapie présentant un transducteur (2) comportant une pluralité d'émetteurs ultrasonores (3) définissant une face d'émission (4) des ondes ultrasonores focalisées présentant un axe de symétrie (S) correspondant à l'axe acoustique ou un plan de symétrie (A1) correspondant au plan acoustique, ces émetteurs ultrasonores étant activés par des signaux délivrés par un générateur de signal faisant partie d'un circuit de commande (7), pour définir une surface de création (8) d'un champ de pression d'ondes ultrasonores focalisées, **caractérisé en ce que** :

   - la surface de création (8) est divisée en au moins N secteurs ($8_1$, $8_2$, ...) (avec N compris entre 2 et 32) pour focaliser les ondes ultrasonores sur des zones focales ($Zc_1$, $Zc_2$, ...) s'établissant dans des plans focaux ($Pf_1$, $Pf_2$, ...), les secteurs de cette surface de création présentant dans un plan de profil ($P_p$), des segments de courbe concave (S1, S2, ...) de longueur finie, asymétriques par rapport au plan de symétrie ($A_1$) ou à l'axe de symétrie (S) ;
   - les centres de courbure ($c_1$, $c_2$, ...) sont asymétriques par rapport au plan (A1) ou à l'axe de symétrie (S) dans la mesure où les centres de courbure sont situés à des distances différentes du plan de symétrie (A1) ou de l'axe de symétrie (S) et/ou à des profondeurs différentes prises selon l'axe de symétrie ;
   - chaque segment de courbe concave (S1, S2, ...) possède un axe propre ($a_1$, $a_2$, ...) passant par un centre de courbure ($c_1$, $c_2$, ...) dudit segment de courbe concave et le milieu dudit segment de courbe concave (S1, S2, ...) ;
   - les axes propres ($a_1$, $a_2$, ...) se coupent entre les zones focales ($Zc_1$, $Zc_2$, ...) et la surface de création (8) ou au-delà des zones focales de manière que les faisceaux issus des secteurs se croisent pour créer une zone focale de recouvrement (Zr) qui est désaxée par rapport au plan de symétrie (A1) ou à l'axe de symétrie (S) et située à distance des plans focaux ($Pf_1$, $Pf_2$, ...), entre les zones focales ($Zc_1$, $Zc_2$, ...) et la surface de création (8) ou au-delà des zones focales ($Zc_1$, $Zc_2$, ...) ;

- les secteurs de cette surface de création (8) sont engendrés soit par la rotation de 2n/N des segments de courbe concave (S1, S2, ...) autour de l'axe de symétrie (S) soit par la translation des segments de courbe (S1, S2, ...) selon une direction (Y) perpendiculaire au plan de profil (Pp) contenant lesdits segments de courbe de manière que les secteurs ($8_1$, $8_2$,...) puissent créer des zones de dépôt d'énergie avec des profils correspondant aux zones focales de recouvrement (Zr);

- les segments de courbe (S1, S2, ...) de la surface de création (8) s'étendent dans le plan de profil (Pp) de part et d'autre de l'axe de symétrie (S) ou du plan de symétrie (A1), en étant disjoints pour permettre le positionnement de la zone focale de recouvrement (Zr) à distance de la surface de création.

2. Appareil de thérapie selon la revendication 1, **caractérisé en ce que** la surface de création (8) est divisée en deux secteurs.

3. Appareil de thérapie selon la revendication 1, **caractérisé en ce que** les bords internes ($8a_1$, $8a_2$, ...) délimitent dans la surface de création (8), un logement pour le montage d'une sonde d'imagerie ultrasonore.

4. Appareil de thérapie selon l'une des revendications 1 à 3, **caractérisé en ce que** la surface de création (8) est issue d'une face (4) définie par les éléments transducteurs, de géométrie torique engendrée par la rotation des segments de courbe concave (S1, S2, ...) autour de l'axe de symétrie (S) de sorte que les segments de courbe concave suivent des arcs de cercles non coïncident qui se coupent de sorte que les zones focales ($Zc_1$, $Zc_2$, ...) possèdent une forme en portions de cercle.

5. Appareil de thérapie selon la revendication 4, **caractérisé en ce que** la face (4) est tronquée de manière symétrique par rapport à l'axe de symétrie (S).

6. Appareil de thérapie selon l'une des revendications 1 à 3, **caractérisé en ce que** la surface de création (8) est issue d'une face (4) définie par les éléments transducteurs, de géométrie cylindrique engendrée par la translation selon une longueur limitée, des deux segments de courbe selon une direction (Y) perpendiculaire au plan de profil (Pp) contenant lesdits segments de courbe de sorte que les zones focales ($Z_1$, $Z_2$, ...) possèdent une forme linéaire.

7. Appareil de thérapie selon l'une des revendications précédentes, **caractérisé en ce que** les émetteurs ultrasonores (3) du transducteur (2) définissent une face d'émission (4) correspondant à la surface de création (8) d'un champ de pression d'ondes ultrasonores focalisées.

8. Appareil de thérapie selon l'une des revendications 1 à 6, **caractérisé en ce que** le générateur de signal faisant partie du circuit de commande (7) est piloté pour délivrer des signaux pour activer les émetteurs ultrasonores (3) répartis en segments, avec une loi de retards ou de phases afin de réaliser la surface de création (8) d'un champ de pression d'ondes ultrasonores focalisées.

9. Appareil de thérapie selon l'une des revendications 1 à 8, **caractérisé en ce que** dans une phase d'exposition, les émetteurs ultrasonores (3) faisant partie d'un secteur ($8_1$, $8_2$, ...) et du secteur symétriquement opposé par rapport à l'axe de symétrie sont activés par des signaux délivrés par le générateur de signal faisant partie du circuit de commande (7) pour créer la zone de dépôt d'énergie correspondante.

10. Appareil de thérapie selon l'une des revendications 1 à 8, **caractérisé en ce que** dans une phase d'exposition, les émetteurs ultrasonores (3) faisant partie d'un secteur ($8_1$, $8_2$, ...) et du secteur symétriquement opposé par rapport au plan de symétrie (A1) sont activés par des signaux délivrés par le générateur de signal faisant partie du circuit de commande (7) pour créer la zone de dépôt d'énergie correspondante d'un côté du plan de symétrie (A1).

11. Appareil de thérapie selon la revendication précédente, **caractérisé en ce que** dans une phase d'exposition subséquente, les émetteurs ultrasonores (3) faisant partie d'un secteur ($8_1$, $8_2$, ...) et du secteur symétriquement opposé par rapport au plan de symétrie (A1) sont activés par des signaux délivrés par le générateur de signal faisant partie du circuit de commande (7) pour créer la zone de dépôt d'énergie correspondante du côté opposé au côté dans lequel est créé la zone de dépôt d'énergie de la phase d'exposition précédente.

12. Appareil de thérapie selon la revendication 10 ou 11, **caractérisé en ce que** les émetteurs ultrasonores (3) sont répartis selon plusieurs secteurs perpendiculaires au plan de symétrie ($A_1$) et **en ce que** les émetteurs ultrasonores des secteurs dans des phases d'exposition successives sont activés par des signaux délivrés par le générateur de signal faisant partie du circuit de commande (7) pour créer des zones de dépôt d'énergie de part et d'autre du plan de symétrie (A1).

**13.** Appareil de thérapie selon l'une des revendications précédentes, **caractérisé en ce que** les émetteurs ultrasonores dans des phases d'exposition successives pour chacune desquelles les centres de courbure ($c_1$, $c_2$, ...) sont situés à des distances différentes du plan de symétrie (A1) ou de l'axe de symétrie (S) et/ou à des profondeurs différentes selon l'axe vertical (Z) sont activés par des signaux délivrés par le générateur de signal faisant partie du circuit de commande (7) de manière à obtenir des zones de dépôt d'énergie désaxées.

**14.** Appareil de thérapie selon la revendication précédente, **caractérisé en ce que** les émetteurs ultrasonores dans des phases d'exposition successives pour lesquelles les centres de courbure ($c_1$, $c_2$, ...) sont situés à des distances différentes du plan de symétrie (A1) ou de l'axe de symétrie (S) et/ou à des profondeurs différentes selon l'axe vertical (Z) sont activés par des signaux délivrés par le générateur de signal faisant partie du circuit de commande (7) de manière à obtenir pour ces phases d'exposition successives, des zones de dépôt d'énergie désaxées de positions différentes avec des tailles identiques ou différentes.

**15.** Appareil de thérapie selon l'une des revendications 9 à 14, **caractérisé en ce que** dans des phases d'exposition successives, les émetteurs ultrasonores sont activés par des signaux délivrés par le générateur de signal faisant partie du circuit de commande (7) de manière que les distances et/ou les profondeurs des centres de courbure sont modifiées d'une phase d'exposition à l'autre afin que les zones de dépôt d'énergie soient concentriques et/ou symétriques et/ou asymétriques et/ou superposées selon l'axe vertical (Z).

**16.** Appareil de thérapie selon l'une des revendications précédentes, **caractérisé en ce que** dans au moins une phase d'exposition complémentaire, les émetteurs ultrasonores (3) sont activés par des signaux délivrés par le générateur de signal faisant partie du circuit de commande (7) de manière à assurer la focalisation des ondes ultrasonores dans des zones focales et à obtenir une zone focale de recouvrement centrée par rapport au plan de symétrie (A1) ou à l'axe de symétrie (S) et située à distance des plans focaux entre les zones focales et la face d'émission (8) ou au-delà des zones focales.

[Fig. 1]

[Fig. 2]

[Fig. 3]

[Fig. 4]

[Fig. 5]

[Fig. 6]

[Fig. 6A]

[Fig. 7A-7C]

$4_1$ $4_4$

$4_2$ $4_3$

X

Y

Z

7A

8

$8_1$ $8_2$

X

Y

Z

7B

8

$8_3$

$8_4$

X

Y

Z

7C

[Fig. 8A]

4

Z

X

Y

[Fig. 8B]

[Fig. 8C]

[Fig. 9A]

[Fig. 9B]

[Fig. 9C]

[Fig. 9D]

[Fig. 10A]

[Fig. 10B]

[Fig. 10C]

[Fig. 10D]

[Fig. 10E]

Zr1 — Zr2

[Fig. 10F]

Zr1 — Zr2

[Fig. 11A]

Zr1 — Zr2

[Fig. 11B]

[Fig. 11C]

[Fig. 11D]

[Fig. 11E]

[Fig. 12]

[Fig. 12A]

[Fig. 12B]

[Fig. 12C]

...

[Fig. 13A]

[Fig. 13B]

[Fig. 13C]

[Fig. 13D]

**Europäisches Patentamt**
**European Patent Office**
**Office européen des brevets**

## RAPPORT DE RECHERCHE EUROPEENNE

Numéro de la demande

EP 21 16 5904

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2011/024074 A2 (INSIGHTEC LTD [IL]; VITEK SHUKI [IL]; VORTMAN KOBI [IL]) 3 mars 2011 (2011-03-03) | 1,7,8,12 | INV. A61N7/00 A61N7/02 |
| Y | * alinéas [0004], [0007], [0009] * <br> * figures 1,3,4 * | 2-6, 9-11, 13-16 | B06B1/00 G10K11/34 |
| Y | WO 2011/092683 A1 (LIVESONICS LTD [IL]; SABBAH BENJAMIN [IL] ET AL.) 4 août 2011 (2011-08-04) * page 22, ligne 28 * * page 23, ligne 1 - ligne 2 * * page 23, ligne 29 - ligne 30 * * figures 6a,6b,6c * | 2,4,5 | ADD. B06B1/02 B06B1/06 G10K11/32 |
| Y | EP 0 661 029 A1 (TOSHIBA KK [JP]) 5 juillet 1995 (1995-07-05) * colonne 4, ligne 54 - colonne 5, ligne 7 * | 3 | |
| Y | US 5 522 869 A (BURDETTE EVERETTE C [US] ET AL) 4 juin 1996 (1996-06-04) * figure 3 * | 6 | DOMAINES TECHNIQUES RECHERCHES (IPC) A61N B06B G10K |
| Y | WO 2016/144931 A1 (UNIV NEW YORK STATE RES FOUND [US]) 15 septembre 2016 (2016-09-15) * alinéa [0030] * * figures 4A,4B,6B * | 9-11, 13-16 | |
| A | US 6 506 171 B1 (VITEK SHUKI [IL] ET AL) 14 janvier 2003 (2003-01-14) * figures 1-6 * | 1-16 | |
| A | EP 2 865 420 A1 (EDAP TMS FRANCE [FR] ET AL.) 29 avril 2015 (2015-04-29) * figures 1-2B * | 1-16 | |

Le présent rapport a été établi pour toutes les revendications

| Lieu de la recherche | Date d'achèvement de la recherche | Examinateur |
|---|---|---|
| La Haye | 21 juillet 2021 | Milles, Julien |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 21 16 5904

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-07-2021

| Document brevet cité au rapport de recherche | Date de publication | Membre(s) de la famille de brevet(s) | Date de publication |
|---|---|---|---|
| WO 2011024074 A2 | 03-03-2011 | US 2011066032 A1<br>WO 2011024074 A2 | 17-03-2011<br>03-03-2011 |
| WO 2011092683 A1 | 04-08-2011 | AUCUN | |
| EP 0661029 A1 | 05-07-1995 | DE 69421256 T2<br>EP 0661029 A1<br>JP H07184907 A<br>US 5643179 A | 16-03-2000<br>05-07-1995<br>25-07-1995<br>01-07-1997 |
| US 5522869 A | 04-06-1996 | US 5522869 A<br>US 5549638 A | 04-06-1996<br>27-08-1996 |
| WO 2016144931 A1 | 15-09-2016 | CN 107427695 A<br>HK 1246228 A1<br>US 2018050223 A1<br>WO 2016144931 A1 | 01-12-2017<br>07-09-2018<br>22-02-2018<br>15-09-2016 |
| US 6506171 B1 | 14-01-2003 | AUCUN | |
| EP 2865420 A1 | 29-04-2015 | CN 104548393 A<br>EP 2865420 A1<br>FR 3012042 A1<br>JP 6419528 B2<br>JP 2015119951 A<br>US 2015112235 A1 | 29-04-2015<br>29-04-2015<br>24-04-2015<br>07-11-2018<br>02-07-2015<br>23-04-2015 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0214782 A
- WO 2011092683 A
- EP 0661029 A
- US 5522869 A

- EP 2035091 A
- EP 2691154 A
- WO 2011024074 A
- WO 2016144931 A

**Littérature non-brevet citée dans la description**

- **KENNEDY, J.E.** High-intensity focused ultrasound in the treatment of solid tumours. *Nat Rev Cancer,* 2005, vol. 5 (4), 321-7
- **AUBRY, J.F. et al.** The road to clinical use of high-intensity focused ultrasound for liver cancer : technical and clinical consensus. *J Ther Ultrasound,* 2013, (1), 13

- **VINCENOT, J. et al.** Electronic beam steering used with a toroidal HIFU transducer substantially increases the coagulated volume. *Ultrasound Med Biol,* 2013, vol. 39 (7), 1241-54